# EUROPEAN PATENT SPECIFICATION

(11) **EP 2 726 060 B1**
(45) Date of publication and mention of the grant of the patent: **25.12.2019**
(21) Application number: 12738675.3
(22) Date of filing: 29.06.2012
(51) Int. Cl.: A61K 9/00, A61K 47/14, A61K 38/13, A61K 31/568, A61K 31/417, A61K 31/4174, A61K 31/5575, A61K 31/5685, A61K 31/165, A61K 31/4025, A61K 31/4164, A61K 9/06, A61K 9/107

(54) **MACROGOL 15 HYDROXYSTEARATE FORMULATIONS**
MACROGOL-15 HYDROXYSTEARAT-FORMULIERUNGEN
FORMULATIONS DE MACROGOL 15 HYDROXYSTÉARATE

(30) Priority: 29.06.2011 US 201161502637 P
(43) Date of publication of application: 07.05.2014
(73) Proprietor: ALLERGAN, INC., Irvine, CA 92612 (US)
(72) Inventor: GORE, Anuradha V., Irvine California 92612 (US); WARNER, Kevin S., Anaheim California 92807 (US); PUJARA, Chetan P., Irvine California 92620 (US); GRAHAM, Richard S., Irvine California 92612 (US); PARASHAR, Ajay P., Irvine California 92612 (US); LEE, Mu-Lan, Tustin California 92782 (US); JORDAN, Robert S., Trabuco Canyon California 92679 (US); LIKITLERSUANG, Sukhon, Irvine California 92602 (US)
(74) Representative: Hoffmann Eitle
(86) International application number: PCT/US2012/045145
(87) International publication number: WO 2013/003827

(56) References cited:
- GAN L ET AL: "Novel microemulsion in situ electrolyte-triggered gelling system for ophthalmic delivery of lipophilic cyclosporine A: In vitro and in vivo results", INTERNATIONAL JOURNAL OF PHARMACEUTICS, ELSEVIER BV, NL, vol. 365, no. 1-2, 5 January 2009 (2009-01-05), pages 143-149, XP025760696, ISSN: 0378-5173, DOI: 10.1016/J.IJPHARM.2008.08.004 [retrieved on 2008-08-15]
- K STREMPEL ET AL: "Effect of Preservation on the Stability of HPMC/Solutol-Stabilized O/W Submicron Emulsions", 4TH WORLD MEETING APGI, FLORENCE, 11 April 2002 (2002-04-11), XP055248955,
- Kibbe ET AL: "Benzalkonium chloride" In: "Handbook of Pharmaceutical Excipients", 1 January 2018 (2018-01-01), XP55503096,

## Description

### CROSS-REFERENCE TO RELATED APPLICATIONS

This application claims the benefit of U.S. Provisional Application No. 61/502,637, filed June 29, 2011.

### BACKGROUND OF THE INVENTION

Topically applied formulations (including formulations applied to the cornea, conjunctiva, eyelid margin, etc) are frequently used in ophthalmology to treat acute and chronic conditions because they are considered to be safer relative to systemically delivered formulations. However, it is often found that the active pharmaceutical ingredients (APIs) intended for topical application may have poor aqueous solubility, thus limiting the maximum dose of drug that can be formulated as a solution. Strategies to increase the solubility of APIs in formulations are thus necessary to achieve the desired dose. Improving solubility is often accomplished by the use of surfactants to improve solution solubility of the drug. Specifically, polyoxyethylated nonionic surfactants, such as Polysorbate 80 (PS80), have been widely used as solubilizers in topically applied formulations of ophthalmic drugs for the treatment of various ocular disorders such as dry eye, inflammation, allergy, ocular hypertension, glaucoma, etc. Therefore, there is a need in the art for formulations that increase the solubility of APIs. Provided herein are compositions addressing these and other needs in the art.

International Journal of Pharmaceutics, vol. 365, 2009, pp. 143-149 discloses a microemulsion comprising cyclosporine A, castor oil, Solutol HS 15, glycerol and water.

### BRIEF SUMMARY OF THE INVENTION

In a first aspect, there is provided an ophthalmic composition which includes an active pharmaceutical ingredient (API) in an amount sufficient to contribute to the treatment, prevention or reduction of a symptom or symptoms of an ophthalmic disease or condition; macrogol 15 hydroxystearate; and benzalkonium chloride at a concentration of 10 to 200 ppm.

In another aspect, there is provided an ophthalmic composition as defined above for use in a method of treating a disease or disorder selected from the group consisting of ocular hypertension, primary open angle glaucoma, ocular inflammation, keratoconjunctivitis sicca, dry eye associated with keratoconjunctivitis sicca, vernel keratoconjunctivitis, atopic keratoconjunctivitis, and corneal insensitivity due to corneal surgery.

### BRIEF DESCRIPTION OF THE DRAWINGS

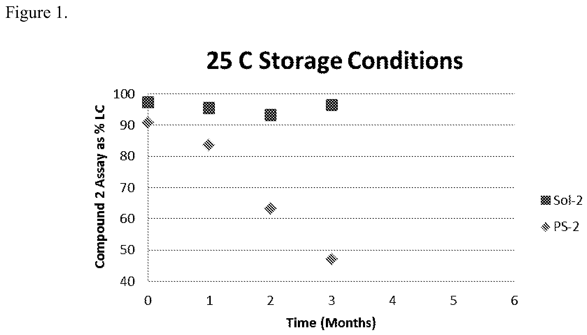

Figure 1. Stability results of Cmpd 2 in ophthalmic formulations shown in Table 2. *See* Examples. Samples were stored at 25°C. Axes: x-axis (time, months); y-axis (stability of Cmpd 2 as measured by percent remaining by liquid chromatography, LC). Legend: macrogol 15 hydroxystearate (box); polysorbate 80 (diamond).

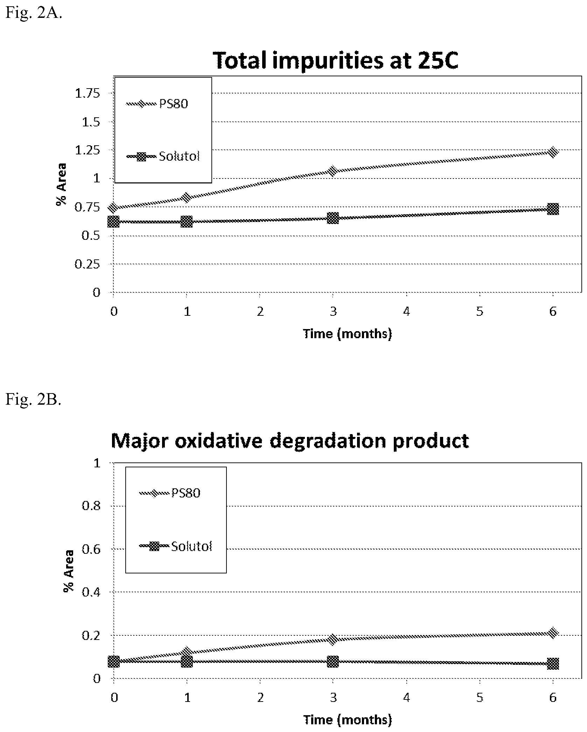

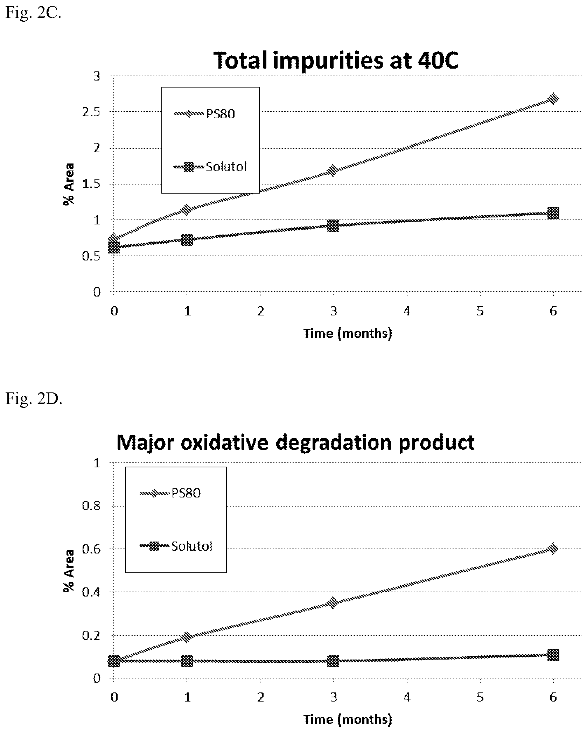

Figure 2: Stability results for Cmpd 1 in formulations containing either polysorbate 80 (PS80) or macrogol 15 hydroxystearate (Sol-2) as solubilizer as shown in Table 3. *See* Examples. Macrogol 15 hydroxystearate was seen to prevent oxidative degradation seen in PS80 containing formulations. Impurity amounts were calculated by percent area by liquid chromatography (LC). Fig. 2A: *25°C* storage, total impurities. Fig. 2B: 25°C storage, major degradation product. Fig. 2C: 40°C storage, total impurities. Fig. 2C: 40°C storage, major degradation product. Legend: as in Figure 1.

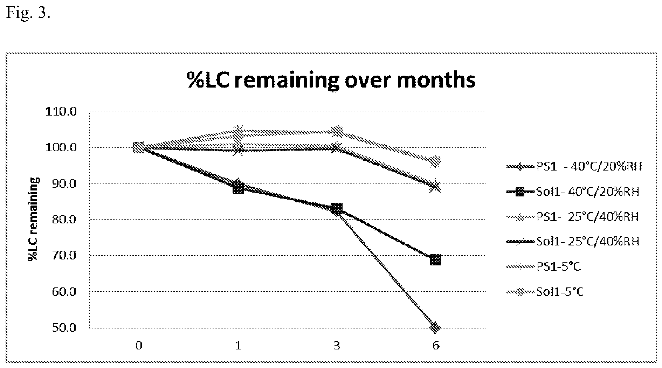

Figure 3: Stability results of Cmpd 2 in formulations shown in Table 7. *See* Examples. Macrogol 15 hydroxystearate was seen to prevent degradation seen in PS80 containing formulations at 40°C/20%RH storage condition. PS1 refers to formulation containing Polysorbate 80 as listed in Table 7. Sol1 refers to formulation containing Macrogol 15 hydroxystearate as listed in Table 7. Axes: x-axis (time, months); y-axis (stability of Cmpd 2 as measured by percent remaining by liquid chromatography, LC). Legend Formulation PS1, storage 40°C/20% RH, % remaining by LC (Diamond); Formulation Sol1, storage 40°C/20% RH, % remaining by LC (Square); Formulation PS1, storage 25°C/40% RH, % remaining by LC (Triangle); Formulation Sol1, storage 25°C/40% RH, % remaining by LC (cross); Formulation PS1, storage 5°C, % remaining by LC (Asterix); Formulation Sol1, storage 5°C, % remaining by LC (circle).

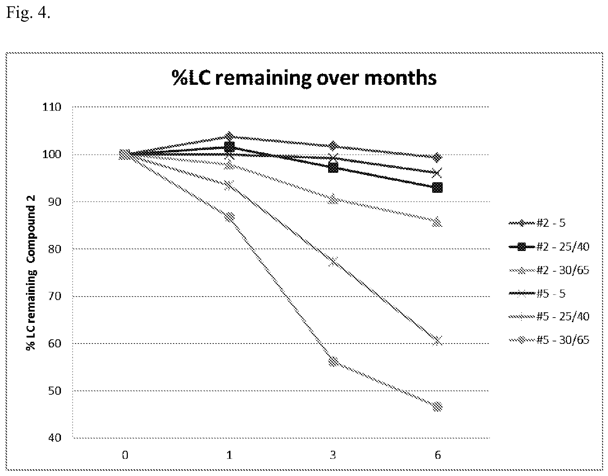

Figure 4: Stability results of Cmpd 2 in formulations shown in Table 8. *See* Examples. #5 refers to formulation containing Polysorbate 80 as listed in Table 8. Macrogol 15 hydroxystearate was seen to prevent degradation seen in PS80 containing formulations at all storage conditions. #2 refers to formulation containing Macrogol 15 hydroxystearate as listed in Table 8. Axes: x-axis (time, months); y-axis (stability of Cmpd 2 as measured by percent remaining by liquid chromatography, LC). Legend Formulation #2, storage 5°, % remaining by LC (Diamond); Formulation #2, storage 25°C/40% RH, % remaining by LC (Square); Formulation #2, storage 30°C/60% RH, % remaining by LC (Triangle); Formulation #5, storage 5°C, % remaining by LC (cross); Formulation #5, storage 25°C/40%RH, % remaining by LC (Asterix); Formulation #5, storage 30°C/60% RH, % remaining by LC (circle).

### DETAILED DESCRIPTION OF THE INVENTION

### I. Definitions

The abbreviations used herein have their conventional meaning within the chemical, biological or pharmaceutical arts. The chemical structures and formulae set forth herein are constructed according to the standard rules of chemical valency known in the chemical arts.

The terms "active pharmaceutical ingredient" and "API" refer to the active ingredient of a drug product. An API is typically a chemical substance or mixture of chemical substances. Such substances are intended to furnish pharmacological activity or other direct effect in the diagnosis, cure, mitigation, treatment or prevention of disease or to effect the structure and function of the body of a subject. "Drug product" refers, in the customary sense, to a composition useful in the diagnosis, cure, mitigation, treatment or prevention of a disease or disorder in the healing arts, e.g., medical or veterinary. Further to any aspect disclosed herein, in some embodiments the composition is a pharmaceutical composition suitable for use as a drug product. "Subject" refers to a mammal, e.g., a human or other animal. "Other animal" in this context refers to non-human mammals (e.g., canine, feline, equine, bovine, caprine).

The term "solubilizing effective amount" of a substance ("solubilizer") within a formulation refers to an amount of the substance sufficient to solubilize another component of the composition. For example, an "API-solubilizing effective amount" is an amount sufficient to solubilize an API such that the API is more therapeutically effective as compared to the absence of the solubilizer. In some embodiments an "API-solubilizing effective amount" is an amount sufficient to solubilize an API such that the API is more therapeutically effective as compared to the absence of the solubilizer in a topical formulation or an ophthalmic formulation.

The term "macrogol 15 hydroxystearate" refers, in the customary sense, to a mixture of mainly monoesters and diesters of 12-hydroxystearic acid and macrogols obtained by the ethoxylation of 12-hydroxystearic acid. Macrogol 15 hydroxystearate is also known as 12-hydroxyoctadecanoic acid polymer with α-hydro-ω-hydroxypoly(oxy-1,2-ethanediyl); 12-hydroxystearic acid polyethylene glycol copolymer; macrogol 15 hydroxystearate; polyethylene glycol-15-hydroxystearate; and polyethylene glycol 660 12-hydroxystearate. In some embodiments, the macrogol 15 hydroxystearate is Solutol® HS 15 (BASF AG, Germany). Solutol® HS 15 consists of polyglycol mono- and di-esters of 12-hydroxystearic acid (i.e., lipophilic part), with about 30% free polyethylene glycol (i.e., hydrophilic part), as known in the art.

The term "emulsifying effective amount" of a substance in a formulation refers to an amount of the substance sufficient to emulsify the composition.

The term "API-preserving effective amount" of a substance in a formulation is an amount of the substance ("preservative") sufficient to preserve an API within the composition. "Preserve" in this context refers, in the customary sense, to the reduction of deterioration or degradation of an API relative to the deterioration in the absence of the preserving substance ("preservative"). Deterioration or degradation may be caused by, for example, time, heat, light or microbiological activity. In some embodiments, the deterioration or degradation of an API is reduced by the preservative by an amount selected from the group consisting of at least 5%, 10%, 15%, 20%, 25%, 30%, 35%, 40%, 45%, 50%, 55%, 60%. 65%, 70%, 75%, 80%, 85%, 90%, 91%, 92%, 93%, 94%, 95%, 96%, 97%, 98%, 99%, and 100% over an amount selected from the group consisting of at least a 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170 and 180 day period.

The term "plant oil" as used herein means a pharmaceutically acceptable oil derived from a plant and includes, for example, anise oil, castor oil, clove oil, cassia oil, cinnamon oil; almond oil, corn oil, arachis oil, cottonseed oil, safflower oil, maize oil, linseed oil, flax seed oil, echium oil, rapeseed oil, soybean oil, olive oil, caraway oil, rosemary oil, peanut oil, peppermint oil, sunflower oil, eucalyptus oil, sesame oil, coriander oil, lavender oil, citronella oil, juniper oil, lemon oil, orange oil, clary sage oil, nutmeg oil, tea tree oil, coconut oil, tallow oil, and lard.

The terms "Carbopol® 980" and "Carbopol® 980 polymer" refer, in the customary sense, to crosslinked polyacrylate polymers as known in the art.

The term "trolamine" refers, in the customary sense, to CAS Registry No. 102-71-6, also known as tris(2-hydroxyethyl)amine, 2,2',2"-trihydroxy-triethylamine, triethylolamine, TEA and TEOA.

The term "medium chain triglyceride" refers, in the customary sense, to medium-chain (e.g., 6 to 12 carbon atoms) fatty acid esters of glycerol. In some embodiments, the medium chain triglyceride includes C₆-C₈ carbon chains.

The term "microemulsion" refers, in the customary sense, to a clear, stable, isotropic liquid mixture of a hydrophobic component (e.g., oil), an aqueous component (e.g., water optionally containing salts and other ingredients), and a surfactant. In contrast to emulsions, microemulsions can form upon simply mixing of the components and do not require the high shear conditions generally used in the formation of emulsions.

The term "lipid nanoparticle" refers, in the customary sense, to a particle of lipophilic compounds which are incorporated into a nanostructured lipid carrier. The resulting lipid nanoparticles may possess a matrix with a controlled structure for optimizing drug incorporation and modifying drug release.

The term "secondary solubilizer" or "solubilizer" in the context of compositions described herein refers to a solubilizer included in addition to macrogol 15 hydroxystearate. Suitable secondary solubilizers include, e.g., sorbitan stearate, polyoxyethylene-polyoxypropylene block copolymer, polyoxyethylene 40 stearate, polyethoxylated castor oil, cyclodextrins, synthetic or semi-synthetic oils (e.g. including as a component, triglycerides (e.g. medium chain triglycerides), triglyceride esters (e.g. triglyceride PEG esters), fatty acids, polyethylene glycols, PEG esters, or mixtures of these and/or other components; Labrafil®, Labrafil® M1944CS, Labrafil® M2125CS, Labrafil® M2130CS, or Labrasol®), Caprylol® 90, Capryol® PGMC, Lauroglycol® 90, Lauroglycol® FCC, Plurol® Oleique CC 497, or Transcutol® P.

"Cyclosporine" refers to the cyclic peptide with systematic name (3S,6S,9S,12R,15S,18S,21S,24S,30S,33S)-30-Ethyl-33-[(1R,2R,4E)-1-hydroxy-2-methyl-4-hexen-1-yl]-6,9,18,24-tetraisobutyl-3,21-diisopropyl-1,4,7,10,12,15,19,25,28-nonamethyl-1,4,7,10,13,16,19,22,25,28,31-undecaazacyclotritriacontane-2,5,8,11,14,17,20,23,26,29,32-undecone, with structure following, including salts and known equivalents thereof. Cyclosporine is also known in the art as, e.g., cyclosporin A, ciclosporin and ciclosporin A.

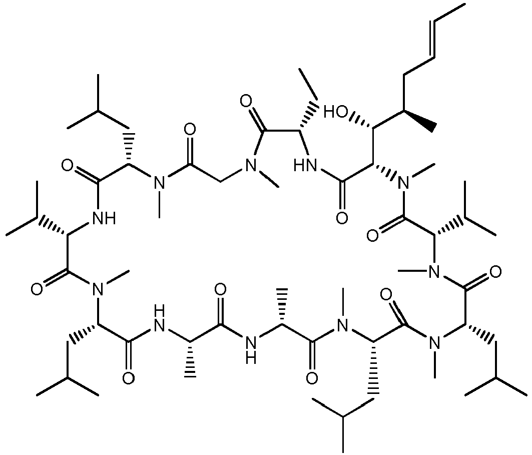

"Simenepag isopropyl" or "Cmpd 1" refers to isopropyl 5-((((R)-1-(4-((S)-1-hydroxyhexyl)phenyl)-5-oxopyrrolidin-2-yl)methoxy)methyl)thiophene-2-carboxylate, with structure following, including salts and known equivalents thereof.

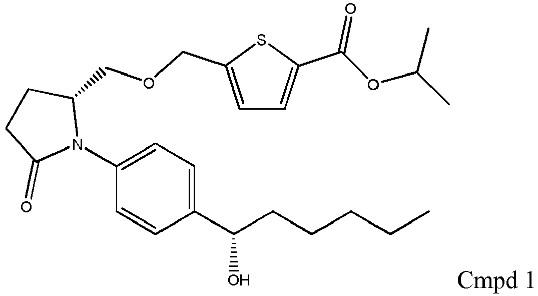

"Aganepag isopropyl" or "Cmpd 2" refers to isopropyl 5-(3-((S)-1-(4-((S)-1-hydroxyhexyl)phenyl)-5-oxopyrrolidin-2-yl)propyl)thiophene-2-carboxylate, with structure following, including salts and known equivalents thereof.

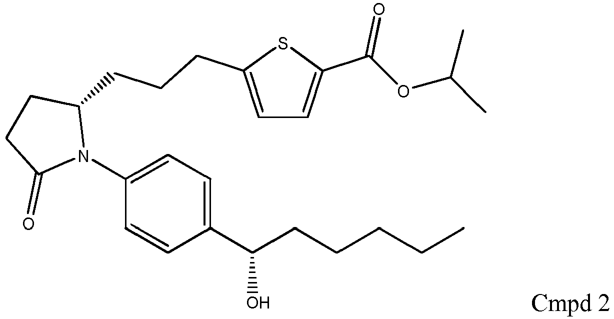

"Cmpd 3" refers to 3-[(1S)-1-(1H-imidazol-4-yl)ethyl]-2-methylbenzyl 2-methylpropanoate, with structure following, including salts and known equivalents thereof.

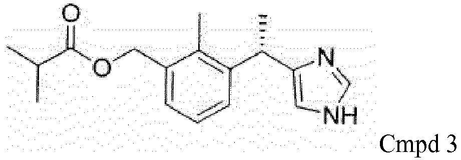

"Cmpd 4" refers to 3-[(1S)-1-(1H-imidazol-4-yl)ethyl]-2-methylbenzylpivalate, with structure following, including salts and known equivalents thereof.

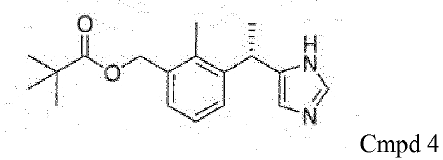

The term "Bimatoprost" refers, in the customary sense, to (Z)-7-((1R,2R,3R,5S)-3,5-dihydroxy-2-((S,E)-3-hydroxy-5-phenylpent-1-enyl)cyclopentyl)-N-ethylhept-5-enamide with structure following, including salts and known equivalents thereof.

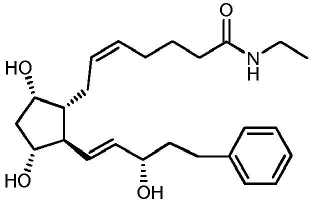

The term "polysorbate 80" refers, in the customary sense, to CAS Registry No. 9005-65-6, also known as polyoxyethylene (80) sorbitan monooleate and sorbitan monooleate ethoxylate, e.g., Alkest TW 80, Tween 80, including salts and known equivalents thereof.

The term "polysorbate 20" refers, in the sense, to CAS Registry No. 9005-64-5, also known as PEG(20) sorbitan monolaurate and polyoxyethylene sorbitan monolaurate, e.g., Alkest TW 20, Tween 20, including salts and known equivalents thereof.

The term "polyoxyethylene 40 stearate" refers, in the customary sense, to polymers of CAS Registry No. 9004-99-3, also known as POE40Stearate, polyoxyl 40 stearate, polyethylene glycol (40) monostearate, polyethylene glycol monostearate, and PEG monostearate, including salts and known equivalents thereof.

The term "sorbitan stearate" refers, in the customary sense, to CAS Registry No. 1338-41-6, also known as sorbitane monostearate, e.g., Span™ 60, including salts and known equivalents thereof.

The term "polyoxyethylene-polyoxypropylene block copolymer" refers, in the customary sense, to polyoxamers of CAS Registry No. 9003-11-6, e.g., Pluronic® F68, including salts and known equivalents thereof.

The terms "polyoxyethylene castor oil" and "castor oil ethoxylated" refer, in the customary sense, to CAS Registry No. 61791-12-6, e.g., Cremophor® EL®, including salts and known equivalents thereof.

The term "capmul" refers, in the customary sense, to a variety of glyceryl esters of e.g., oleate, sterarate, laurate, and caprate.

The term "stabilized oxychloro complex" refers, in the customary sense, to an equilibrium mixture of oxychloro species, predominantly chlorite (NaClO₂), chlorate (NaClO₃) and traces of chlorine dioxide (ClO₂), e.g., Purite®, including salts and known equivalents thereof.

The term "HPMC" refers, in the customary sense, to hydroxypropyl methycellulose, e.g., HPMC E4M and HPMC F4M, as known in the art, including salts and known equivalents thereof.

An "effective amount" is an amount sufficient to contribute to the treatment, prevention, or reduction of a symptom or symptoms of a disease or condition. An "effective amount" may also be referred to as a "therapeutically effective amount." An "ophthalmically effective amount" is an amount sufficient to contribute to the treatment, prevention, or reduction of a symptom or symptoms of an ophthalmic disease or condition.

The term "consisting essentially of' or "consists essentially of' means consisting of the named components or listed items and any additional unnamed components or unlisted items that would not cause the function of the composition (e.g. a function set forth in the methods disclosed herein) containing the named and unnamed components to be materially different from a composition consisting of only the named components.

### II. Compositions

The composition of the invention includes an active pharmaceutical ingredient (API) and macrogol 15 hydroxystearate. In some embodiments, the macrogol 15 hydroxystearate is present in an API-solubilizing effective amount. In some embodiments, the macrogol 15 hydroxystearate is not present in an emulsifying effective amount. In some embodiments, the composition is not an emulsion. In some embodiments, the macrogol 15 hydroxystearate is present in an emulsifying effective amount. In some embodiments, the composition is an emulsion.

In some embodiments, the composition does not include a plant oil. In some embodiments, the composition includes a plant oil, e.g., castor oil. In some embodiments, the composition includes a plant oil which has been further chemically modified, e.g., polyethoxylated castor oil.

In some embodiments, the API is selected from the group consisting of cyclosporine, phentolamine, testosterone, testosterone derivative, simenepag isopropyl, aganepag isopropyl, Cmpd 3, Cmpd 4, or bimatoprost, and pharmaceutically acceptable salts thereof. In some embodiments, the API is cyclosporine. In some embodiments, the API is phentolamine. In some embodiments, the API is testosterone. In some embodiments, the API is a testosterone derivative. In some embodiments, the API is simenepag isopropyl. In some embodiments, the API is aganepag isopropyl. In some embodiments, the API is Cmpd 3. In some embodiments, the API is Cmpd 4. In some embodiments, the API is bimatoprost. In some embodiments, the API is a pharmaceutically acceptable salt of a specific API provided herein.

In some embodiments, the composition includes cyclosporine at a concentration of 0.001 to 0.1%(w/w). It is understood that, absent express indication otherwise, the term "at a concentration of' is inclusive for the indicated range. For example, the term "at a concentration of 0.001 to 0.1%(w/w)" means 0.001% (w/w), 0.1% (w/w), and all concentrations between 0.001% (w/w) and 0.1% (w/w). In some embodiments, the composition includes cyclosporine at a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, and 0.1%(w/w).

In some embodiments, the composition includes phentolamine at a concentration of 0.001 to 1.0%(w/w). In some embodiments, the composition includes phentolamine at a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w). In some embodiments, the composition includes testosterone at a concentration of 0.001 to 5.0%(w/w). In some embodiments, the composition includes testosterone at a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w). In some embodiments, the composition includes a testosterone derivative at a concentration of 0.001 to 5.0%(w/w). In some embodiments, the composition includes a testosterone derivative at a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w). In some embodiments, the composition includes simenepag isopropyl at a concentration of 0.001 to 2.5%(w/w). In some embodiments, the composition includes simenepag isopropyl at a concentration of 0.001 to 0.1%(w/w). In some embodiments, the composition includes simenepag isopropyl at a concentration selected from the group consisting of about 0.0001, 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w). In some embodiments, the composition includes aganepag isopropyl at a concentration of 0.001 to 2.5%(w/w). In some embodiments, the composition includes aganepag isopropyl at a concentration of 0.001 to 0.1%(w/w). In some embodiments, the composition includes aganepag isopropyl at a concentration of 0.0002 to 0.05%(w/w). In some embodiments, the composition includes aganepag isopropyl at a concentration selected from the group consisting of about 0.0001, 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w). In some embodiments, the composition includes Cmpd 3 at a concentration of 0.001 to 2.5%(w/w). In some embodiments, the composition includes Cmpd 3 at a concentration of 0.001 to 0.1%(w/w). In some embodiments, the composition includes Cmpd 3 at a concentration selected from the group consisting of about 0.0001, 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w). In some embodiments, the composition includes Cmpd 4 at a concentration of 0.001 to 2.5%(w/w). In some embodiments, the composition includes Cmpd 4 at a concentration of 0.001 to 0.1%(w/w). In some embodiments, the composition includes Cmpd 4 at a concentration selected from the group consisting of about 0.0001, 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w). In some embodiments, the composition includes bimatoprost at a concentration of 0.001 to 2.5%(w/w). In some embodiments, the composition includes bimatoprost at a concentration of 0.001 to 0.1%(w/w). In some embodiments, the composition includes bimatoprost at a concentration selected from the group consisting of about 0.0001, 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w).

The composition further includes benzalkonium chloride at a concentration of 10 to 200 ppm. In some embodiments, the benzalkonium chloride is present in an API-preserving effective amount that is reduced relative to a composition not including macrogol 15 hydroxystearate. In some embodiments, the API-preserving effective amount is reduced relative to a composition not including macrogol 15 hydroxystearate and including a substance selected from the group consisting of polysorbate 80, polysorbate 20 and polyoxyethylene 40 stearate. In some embodiments, the macrogol 15 hydroxystearate is present in an API solubilizing effective amount.

Further to any embodiment above, in some embodiments the macrogol 15 hydroxystearate is present at a concentration of 0.1 to 50%(w/w). In some embodiments the macrogol 15 hydroxystearate is present at a concentration of 0.1 to 25%(w/w). In some embodiments the macrogol 15 hydroxystearate is present at a concentration of 0.1 to 10%(w/w). Further to any embodiment above, in some embodiments the macrogol 15 hydroxystearate is present at a concentration of 0.1 to 5%(w/w). In some embodiments the macrogol 15 hydroxystearate is present at a concentration of 0.1 to 1.0%(w/w). In some embodiments the macrogol 15 hydroxystearate is present at a concentration of 0.01 to 1.0%(w/w). In some embodiments the macrogol 15 hydroxystearate is present at a concentration of 0.01 to 0.1%(w/w). In some embodiments the macrogol 15 hydroxystearate is present at a concentration of 0.001 to 0.01%(w/w). In some embodiments the macrogol 15 hydroxystearate is present at a concentration selected from the group consisting of 0.1 to 2.0%(w/w)%(w/w), 0.2 to 2.0%(w/w)%(w/w), 0.3 to 2.0%(w/w), 0.4 to 2.0%(w/w), 0.5 to 2.0%(w/w), 0.6 to 2.0%(w/w), 0.7 to 2.0%(w/w), 0.8 to 2.0%(w/w), 0.9 to 2.0%(w/w), 1.0 to 2.0%(w/w), 1.1 to 2.0%(w/w), 1.2 to 2.0%(w/w), 1.3 to 2.0%(w/w), 1.4 to 2.0%(w/w), 1.5 to 2.0%(w/w), 1.6 to 2.0%(w/w), 1.7 to 2.0%(w/w), 1.8 to 2.0%(w/w), 1.9 to 2.0%(w/w), 0.1 to 1.9%(w/w), 0.1 to 1.8%(w/w), 0.1 to 1.7%(w/w), 0.1 to 1.6%(w/w), 0.1 to 1.5%(w/w), 0.1 to 1.4%(w/w), 0.1 to 1.3%(w/w), 0.1 to 1.2%(w/w), 0.1 to 1.1%(w/w), 0.1 to 1.0%(w/w), 0.1 to 0.9%(w/w), 0.1 to 0.8%(w/w), 0.1 to 0.7%(w/w), 0.1 to 0.6%(w/w), 0.1 to 0.5%(w/w), 0.1 to 0.4%(w/w), 0.1 to 0.3%(w/w), 0.1 to 0.2%(w/w), 0.2 to 1.9%(w/w), 0.3 to 1.8%(w/w), 0.4 to 1.7%(w/w), 0.5 to 1.6%(w/w), 0.6 to 1.5%(w/w), 0.7 to 1.4%(w/w), 0.8 to 1.3%(w/w), 0.9 to 1.2%(w/w), and 0.9 to 1.1%(w/w). In some embodiments, the composition is an ointment. In some embodiments, the macrogol 15 hydroxystearate is present at a concentration of 0.1 to 3%(w/w). In some embodiments, the composition is a cream. In some embodiments, the macrogol 15 hydroxystearate is present at a concentration of about 0.67%(w/w). In some embodiments, the composition is a microemulsion. Unless indicated otherwise, the term "about" in the context of a numeric value indicates the nominal value ± 10% of the nominal value. In some embodiments, the macrogol 15 hydroxystearate is present at a concentration of 0.01 to 5%(w/w). In some embodiments, the composition includes lipid nanoparticles. In some embodiments, the macrogol 15 hydroxystearate is present at a concentration of 0.01 to 2%(w/w). In some embodiments, the composition is an emulsion. Further to any embodiment above, in some embodiments the macrogol 15 hydroxystearate is present at a concentration of about 1.0%(w/w). In some embodiments, the macrogol 15 hydroxystearate is present at a concentration of 0.001 to 5%(w/w). In some embodiments, the macrogol 15 hydroxystearate is present at a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 6.0, 7.0, 8.0, 9.0, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50%(w/w). In some embodiments the composition includes benzalkonium chloride at a concentration of 0.005 to 0.02%(w/w) (e.g. a concentration selected from the group consisting of about 0.005, 0.006, 0.007, 0.008, 0.009, 0.01, 0.011, 0.012, 0.013, 0.014, 0.015, 0.016, 0.017, 0.018, 0.019, and 0.02%(w/w)). In some embodiments the composition includes HPMC at a concentration of 0.25 to 1.0% (w/w) (e.g. a concentration selected from the group consisting of about 0.25, 0.3, 0.35, 0.4, 0.45, 0.5, 0.55, 0.6, 0.65, 0.7, 0.75, 0.8, 0.85. 0.9, and 1.0%(w/w)). In some embodiments the composition includes propylene glycol at a concentration of 2 to 20% (w/w) (e.g. a concentration selected from the group consisting of about 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). In some embodiments the composition includes benzyl alcohol at a concentration of 1 to 5% (w/w) (e.g. a concentration selected from the group consisting of about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments the composition includes isopropyl myristate at a concentration of 10 to 25% (w/w) (e.g. a concentration selected from the group consisting of about 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, and 25%(w/w)). In some embodiments the composition includes Carbopol® 980 at a concentration of 0.1 to 2% (w/w) (e.g. a concentration selected from the group consisting of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments the composition includes petrolatum at a concentration of 20 to 30% (w/w) (e.g. a concentration selected from the group consisting of about 20, 20.5, 21, 21.5, 22, 22.5, 23, 23.5, 24, 24.5, 25, 25.5, 26, 26.5, 27, 27.5, 28, 28.5, 29, 29.5, and 30%(w/w)). In some embodiments the composition includes stearyl alcohol at a concentration of 1 to 30% (w/w) (e.g. a concentration selected from the group consisting of about 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, and 30%(w/w)). In some embodiments the composition includes stearic acid at a concentration of 10 to 15% (w/w) (e.g. a concentration selected from the group consisting of about 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6, 13.7, 13.8, 13.9, 14.0, 14.1, 14.2, 14.3, 14.4, 14.5, 14.6, 14.7, 14.8, 14.9, and 15.0%(w/w)). In some embodiments the composition includes cetyl alcohol at a concentration of 1 to 3% (w/w) (e.g. a concentration selected from the group consisting of about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0%(w/w)). In some embodiments the composition includes medium chain triglycerides at a concentration of 10 to 40% (w/w) (e.g. a concentration selected from the group consisting of about 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, and 40%(w/w)). In some embodiments the composition includes oleic acid at a concentration of 0 to 0.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, and 0.5%(w/w)). In some embodiments the composition includes castor oil at a concentration of 0.1 to 1.25% (w/w) (e.g. a concentration selected from the group consisting of about 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.05, 1.1, 1.15, 1.2, and 1.25%(w/w)). In some embodiments the composition includes glycerin at a concentration of 8-12% (w/w) (e.g. a concentration selected from the group consisting of about 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, and 12.0%(w/w)).

In another aspect, there is provided a composition including cyclosporine, macrogol 15 hydroxystearate, benzalkonium chloride at a concentration of 10 to 200 ppm, an osmolality agent, and a buffer. In some embodiments, cyclosporine is present at a concentration of 0.001-0.1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, and 0.1%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

There is also provided a composition which consists essentially of cyclosporine, macrogol 15 hydroxystearate, an osmolality agent, a buffer, a secondary solubilizer and a preservative which is benzalkonium chloride at a concentration of 10 to 200 ppm. In some embodiments, cyclosporine is present at a concentration of 0.001-0.1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, and 0.1%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In some embodiments there is provided a composition which consists of cyclosporine, macrogol 15 hydroxystearate, an osmolality agent, a buffer, a secondary solubilizer and a preservative which is benzalkonium chloride at a concentration of 10 to 200 ppm. In some embodiments, cyclosporine is present at a concentration of 0.001-0.1% (w/w) (e.g. about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.1%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including phentolamine, macrogol 15 hydroxystearate, benzalkonium chloride at a concentration of 10 to 200 ppm, an osmolality agent, and a buffer. In some embodiments, phentolamine is present at a concentration of 0.001-1.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

There is also provided a composition which consists essentially of phentolamine, macrogol 15 hydroxystearate, an osmolality agent, a buffer, a secondary solubilizer and a preservative which is benzalkonium chloride at a concentration of 10 to 200 ppm. In some embodiments, phentolamine is present at a concentration of 0.001-1.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In other embodiments there is provided a composition which consists of phentolamine, macrogol 15 hydroxystearate, an osmolality agent, a buffer, a secondary solubilizer and a preservative which is benzalkonium chloride at a concentration of 10 to 200 ppm. In some embodiments, phentolamine is present at a concentration of 0.001-1.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including testosterone, macrogol 15 hydroxystearate, benzalkonium chloride at a concentration of 10 to 200 ppm, an osmolality agent, and a buffer. In some embodiments, testosterone is present at a concentration of 0.001-5.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In certain embodiments there is provided a composition which consists essentially of testosterone, macrogol 15 hydroxystearate, an osmolality agent, a buffer, a secondary solubilizer and a preservative which is benzalkonium chloride at a concentration of 10 to 200 ppm. In some embodiments, testosterone is present at a concentration of 0.001-5.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another embodiment there is provided a composition which consists of testosterone, macrogol 15 hydroxystearate, an osmolality agent, a buffer, a secondary solubilizer and a preservative which is benzalkonium chloride at a concentration of 10 to 200 ppm. In some embodiments, testosterone is present at a concentration of 0.001-5.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including a testosterone derivative, macrogol 15 hydroxystearate, benzalkonium chloride at a concentration of 10 to 200 ppm, an osmolality agent, and a buffer. In some embodiments the testosterone derivative is present at a concentration of 0.001-5.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists essentially of a testosterone derivative, macrogol 15 hydroxystearate, an osmolality agent, a buffer, a secondary solubilizer and a preservative which is benzalkonium chloride at a concentration of 10 to 200 ppm. In some embodiments, the testosterone derivative is present at a concentration of 0.001-5.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

There is also provided a composition which consists of a testosterone derivative, macrogol 15 hydroxystearate, an osmolality agent, a buffer, a secondary solubilizer and a preservative which is benzalkonium chloride at a concentration of 10 to 200 ppm. In some embodiments, the testosterone derivative is present at a concentration of 0.001-5.0% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including simenepag isopropyl, macrogol 15 hydroxystearate, benzalkonium chloride at a concentration of 10 to 200 ppm, an osmolality agent, and a buffer. In some embodiments simenepag isopropyl is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, simenepag isopropyl is present at a concentration of 0.001 to 2.5%(w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists essentially of simenepag isopropyl, macrogol 15 hydroxystearate, an osmolality agent, a buffer, a secondary solubilizer and a preservative which is benzalkonium chloride at a concentration of 10 to 200 ppm. In some embodiments simenepag isopropyl is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, simenepag isopropyl is present at a concentration of 0.001 to 2.5%(w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists of simenepag isopropyl, macrogol 15 hydroxystearate, an osmolality agent, a buffer, a secondary solubilizer and a preservative which is benzalkonium chloride at a concentration of 10 to 200 ppm. In some embodiments simenepag isopropyl is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, simenepag isopropyl is present at a concentration of 0.001 to 2.5%(w/w) (e.g. a concentration selected from the group consisting of about 0. 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including aganepag isopropyl, macrogol 15 hydroxystearate, benzalkonium chloride at a concentration of 10 to 200 ppm, an osmolality agent, and a buffer. In some embodiments aganepag isopropyl is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, aganepag isopropyl is present at a concentration of 0.001 to 2.5%(w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments, aganepag isopropyl is present at a concentration of 0.0002 to 0.05%(w/w) (e.g. a concentration selected from the group consisting of about 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, and 0.05%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists essentially of aganepag isopropyl, macrogol 15 hydroxystearate, an osmolality agent, a buffer, a secondary solubilizer and a preservative which is benzalkonium chloride at a concentration of 10 to 200 ppm. In some embodiments aganepag isopropyl is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, aganepag isopropyl is present at a concentration of 0.001 to 2.5%(w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments, aganepag isopropyl is present at a concentration of 0.0002 to 0.05%(w/w) (e.g. a concentration selected from the group consisting of about 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, and 0.05%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

Yet further to this aspect, in one embodiment there is provided a composition which consists of aganepag isopropyl, macrogol 15 hydroxystearate, an osmolality agent, a buffer, a secondary solubilizer and a preservative which is benzalkonium chloride at a concentration of 10 to 200 ppm. In some embodiments aganepag isopropyl is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, aganepag isopropyl is present at a concentration of 0.001 to 2.5%(w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments, aganepag isopropyl is present at a concentration of 0.0002 to 0.05%(w/w) (e.g. a concentration selected from the group consisting of about 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, and 0.05%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including Cmpd 3, macrogol 15 hydroxystearate, benzalkonium chloride at a concentration of 10 to 200 ppm, an osmolality agent, and a buffer. In some embodiments, Cmpd 3 is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments Cmpd 3 is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Sstabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists essentially of Cmpd 3, macrogol 15 hydroxystearate, an osmolality agent, a buffer, a secondary solubilizer and a preservative which is benzalkonium chloride at a concentration of 10 to 200 ppm. In some embodiments, Cmpd 3 is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments Cmpd 3 is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

Yet further to this aspect, in one embodiment there is provided a composition which consists of Cmpd 3, macrogol 15 hydroxystearate, an osmolality agent, a buffer, a secondary solubilizer and a preservative which is benzalkonium chloride at a concentration of 10 to 200 ppm. In some embodiments, Cmpd 3 is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments Cmpd 3 is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including Cmpd 4, macrogol 15 hydroxystearate, benzalkonium chloride at a concentration of 10 to 200 ppm, an osmolality agent, and a buffer. In some embodiments, Cmpd 4 is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments Cmpd 4 is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm. Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists essentially of Cmpd 4, macrogol 15 hydroxystearate, an osmolality agent, a buffer, a secondary solubilizer and a preservative which is benzalkonium chloride at a concentration of 10 to 200 ppm. In some embodiments, Cmpd 4 is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments Cmpd 4 is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

Yet further to this aspect, in one embodiment there is provided a composition which consists of Cmpd 4, macrogol 15 hydroxystearate, an osmolality agent, a buffer, a secondary solubilizer and a preservative which is benzalkonium chloride at a concentration of 10 to 200 ppm. In some embodiments, Cmpd 4 is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments Cmpd 4 is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In another aspect, there is provided a composition including bimatoprost, macrogol 15 hydroxystearate, benzalkonium chloride at a concentration of 10 to 200 ppm, an osmolality agent, and a buffer. In some embodiments, bimatoprost is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments bimatoprost is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol, glycerin, mannitol, and sodium chloride. In some embodiments, propylene glycol is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin is present at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and/or sodium chloride is present at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). Unless indicated otherwise, it is understood that the term "up to" in the context of a concentration is inclusive; i.e., "up to 2%" means zero to 2% (inclusive). In some embodiments, the buffer is a buffer selected from the group consisting of phosphate, phosphate citrate, sodium hydroxide/trolamine, lactate, borate and borate citrate, as known in the art. In some embodiments, phosphate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), or borate citrate is present at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM).

In some embodiments a secondary solubilizer is provided. In some embodiments, the secondary solubilizer is one or more secondary solubilizers selected from the group consisting of of sorbitan stearate, polyoxyethylene-polyoxypropylene block copolymer, polyoxyethylene 40 stearate, polyethoxylated castor oil, and cyclodextrins. In some embodiments, sorbitan stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer is present at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil is present at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and/or cyclodextrins are present at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)).

Benzalkonium chloride is present at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, and 200 ppm). Stabilized oxychloro complex may additionally be present at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

In one embodiment there is provided a composition which consists essentially of bimatoprost, macrogol 15 hydroxystearate, an osmolality agent, a buffer, a secondary solubilizer and a preservative which is benzalkonium chloride at a concentration of 10 to 200 ppm. In some embodiments, bimatoprost is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments bimatoprost is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

Yet further to this aspect, in one embodiment there is provided a composition which consists of bimatoprost, macrogol 15 hydroxystearate, an osmolality agent, a buffer, a secondary solubilizer and a preservative which is benzalkonium chloride at a concentration of 10 to 200 ppm. In some embodiments, bimatoprost is present at a concentration of 0.001-2.5% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)). In some embodiments bimatoprost is present at a concentration of 0.001-0.1% (w/w) (e.g. a concentration selected from the group consisting of about 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, and 0.1%(w/w)). In some embodiments, macrogol 15 hydroxystearate is present at a concentration of 0.001-5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)). In some embodiments, the osmolality agent is one or more osmolality agents selected from the group consisting of propylene glycol at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)), glycerin at a concentration up to 2.5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, and 2.5%(w/w)), mannitol at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), and sodium chloride at a concentration up to 2% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, and 2.0%(w/w)). In some embodiments, the buffer is a buffer selected from the group consisting of phosphate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), phosphate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), sodium hydroxide/trolamine at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), borate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM), and borate citrate at a concentration of 1-100 mM (e.g. a concentration selected from the group consisting of about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, and 100mM). In some embodiments, the secondary solubilizer is one or more secondary solubilizers selected from the group consisting of sorbitan stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyoxyethylene-polyoxypropylene block copolymer at a concentration up to 5% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0%(w/w)), polyoxyethylene 40 stearate at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), polyethoxylated castor oil at a concentration up to 1% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, and 1.0%(w/w)), and cyclodextrins at a concentration up to 10% (w/w) (e.g. a concentration selected from the group consisting of about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.5, 6.0, 6.5, 7.0, 7.5, 8.0, 8.5, 9.0, 9.5, and 10.0%(w/w)). The preservative is benzalkonium chloride at a concentration of 10-200 ppm (e.g. a concentration selected from the group consisting of about 10, 20, 30, 40, 50, 60, 70, 80, 90, 100, 110, 120, 130, 140, 150, 160, 170, 180, 190, or 200 ppm), and optionally stabilized oxychloro complex at a concentration of 10-300 ppm (e.g. a concentration selected from the group consisting of about 10, 20, 30, 40, 50, 60, 70, 80, 90, 100, 110, 120, 130, 140, 150, 160, 170, 180, 190, 200, 210, 220, 230, 240, 250, 260, 270, 280, 290, and 300 ppm).

The terms "composition" and "formulation" are used herein interchangeably and generally refer to ophthalmically acceptable compositions and formulations. Thus, the compositions and formulations provided herein may include additional ingredients generally known in the pharmaceutical arts as needed. For example, tonicity agents may be added to the compositions of the invention as needed. They include, but are not limited to, salts, particularly sodium chloride, potassium chloride, mannitol and glycerin, or any other suitable ophthalmically acceptable tonicity adjustor. In one embodiment, the tonicity agent is present in an amount of between about 0.1 % (w/v) and about 10% (w/v). In another embodiment, the tonicity agent is present in an amount of between about 1.0% and 1.2%. The vehicle for the composition may be saline, water, or some other physiologically compatible vehicle. The composition may be maintained at a comfortable pH with an appropriate buffer system. A desirable pH may be 7.4 - 7.6. Various buffers and means for adjusting pH may be used so long as the resulting preparation is ophthalmically acceptable. Accordingly, buffers include, but are not limited to, acetate buffers, citrate buffers, phosphate buffers and borate buffers. Acids or bases may be used to adjust the pH of these formulations as needed. In one embodiment, the buffer is boric acid at a concentration of between about 0.6% (w/v) and about 0.7% (w/v).

The compositions and formulations provided herein include an ophthalmically effective amount (i.e. a therapeutically effective amount) of the API.

### III. Methods

Any references in the description to treatments or to methods of treatment refer to the compositions or formulations of the present invention for use in a method of treatment.

In another aspect, there is provided a composition as disclosed above for use in a method of treating a disease or disorder selected from the group consisting of ocular hypertension, primary open angle glaucoma, ocular inflammation, keratoconjunctivitis sicca, dry eye associated with keratoconjunctivitis sicca, vernel keratoconjunctivitis, atopic keratoconjunctivitis, and corneal insensitivity due to corneal surgery.

In some embodiments, the method further includes co-administering another active pharmaceutical ingredient to the subject. Exemplary active pharmaceutical ingredients for co-administration include antimicrobials and anti-inflammatories (e.g., steroids and non-steroidal anti-inflammatories), as known in the medical and veterinary arts.

In some embodiments, the disease or disorder is ocular hypertension. In some embodiments, the disease or disorder is primary open angle glaucoma. In some embodiments, the disease or disorder is ocular inflammation. In some embodiments, the disease or disorder is keratoconjunctivitis sicca. In some embodiments, the disease or disorder is dry eye associated with keratoconjunctivitis sicca. In some embodiments, the disease or disorder is vernel keratoconjunctivitis. In some embodiments, the disease or disorder is atopic keratoconjunctivitis. In some embodiments, the disease or disorder is corneal insensitivity due to corneal surgery.

### IV. Formulation development

Without being bound by any particular theory, formulation development activities utilizing polyoxyethylated surfactants (i.e., Polysorbate 80, Polysorbate 20, Polyoxyl stearate 40) uncovered the following interactions between the surfactant, other formulation excipients, and the drug:
1. Oxidative degradation of the drug substance.
2. Degradation of Polysorbate 80 (via auto-oxidation) resulting in changes in physical chemical properties of the surfactant.
3. Reduced benzalkonium chloride (preservative) effectiveness. Benzalkonium chloride (BAK) interaction with the micelles of the surfactant reduces the free BAK available for preservative efficacy.
4. Reduced permeability/bioavailability of API through biological membranes presumably due to a fraction being sequestered in the surfactant micelles.

Provided herein, *inter alia*, are topical ophthalmic formulations containing the polyethylene glycol fatty ester surfactant Solutol® 15 HS for application to the cornea surface of the eye. Solutol® 15 HS is a non-ionic surfactant which can be used both as solubilizer or emulsifier.

Formulations containing Solutol® HS 15 as surfactant in place of the polyoxyethylated surfactants were observed to show several advantages. These include the following.
1. Solubility enhancement of APIs which is superior or comparable to that of polyoxyethylated surfactants.
2. Improved stability of APIs susceptible to degradation by oxidation mechanisms.
3. Improved preservative effectiveness of BAK.
4. Stability of Solutol® 15 HS as it does not undergo auto-oxidation.
5. Better efficacy of API observed, presumably due to better permeability/bioavailability from formulations
6. Improved tolerability for ophthalmic use.

The examples in following tables and figures illustrate embodiments of the invention. However, it is to be understood that the following are only exemplary or illustrative of the application of the principles of the present invention. No specific limitation is imposed on the type of topical drug delivery formulation so long as the formulation has a composition that contains excipients/components consistent with dermal drug delivery, improve the BAK efficacy, drug and formulation stability. Numerous modifications and alternative compositions, methods, and systems may be devised by those skilled in the art.

### V. Examples

### Example 1 (reference example) -- Solubilizing capacity with Solutol® 15 HS as compared to polyoxyethylated surfactants

Table 1 summarizes solubility of Cmpd 1 in vehicles with 4 different solubilizers over a range of temperatures. Solubility of Cmpd 1 in vehicle containing Solutol® 15 HS is higher than that containing POE40 stearate or polysorbate 20, but slightly lower than that containing Polysorbate 80.

**Table 1: Cmpd 1 Solubility in four different formulation vehicles at different temperatures**

| **Solubilizer used (conc. of solubilizer is 1%** | **Temperature (°C)** | **Solubility (mg/ml) measured after 8 weeks** |
|---|---|---|
| Solutol® HS 15 | 5 | 1.502 |
| | 25 | 1.320 |
| | 40 | 1.237 |
| Polysorbate 20 | 5 | 1.242 |
| | 25 | 1.178 |
| | 40 | 1.050 |
| POE 40 Stearate | 5 | 1.268 |
| | 25 | 1.213 |
| | 40 | 1.149 |
| Polysorbate 80 | 5 | 1.678 |
| | 25 | 1.524 |
| | 40 | 1.457 |

Examples of other compounds which show comparable or better solubility in formulations containing Solutol® 15 HS as compared to polyoxyethylated surfactants include Cmpd 2.

### Example 2 -- Improved drug stability when Solutol® 15 HS is used as surfactant.

Improved stability of Cmpd 2 and Cmpd 1 was observed in solutions containing Solutol® HS 15 as a solubilizer as compared to that of polysorbate 80. *See* Tables 2-3 following.

**Table 2: Formulation composition of ophthalmic solutions containing the active Cmpd 2 and Polysorbate 80 and Solutol® HS 15, respectively as solubilizers.**

| **Formulation** # | **1 (Control)** | **2** |
|---|---|---|
| **Ingredients** | **% (w/w)** | **% (w/w)** |
| Cmpd 2 | 0.0025 | 0.0025 |
| HPMC (F4M) | 1.0 | 1.0 |
| BAK | 0.012 | 0.012 |
| Polysorbate 80 | 0.05 | -- |
| Solutol® HS 15 | -- | 0.05 |
| Citric Acid Monohydrate | 0.03 | 0.03 |
| Sodium Phosphate Dibasic Heptahydrate | 0.3 | 0.3 |
| Glycerin | 2.2 | 2.2 |
| EDTA | 0.01 | 0.01 |
| Water for Injection | q. s. to 100% | q.s. to 100% |

**Table 3: Formulation composition of ophthalmic solutions containing the active Cmpd 1 and Polysorbate 80 and Solutol® HS 15, respectively as solubilizers.**

| **Ingredients** | **1 (Control)** | **2** |
|---|---|---|
| | **%w/w** | **%w/w** |
| Cmpd 1 | 0.075 | 0.075 |
| Benzalkonium Chloride | 0.0200 | 0.0200 |
| Edetate Disodium | 0.01 | 0.01 |
| Polysorbate 80 (Super Refined, R18674) | 1.0 | - |
| Solutol® HS 15 (Polyethylene glycol- 15-h hydroxystearate) | -- | 1.0 |
| Polysorbate 20, Super Refined (Croda) | -- | -- |
| POE 40 Stearate (Polyoxyethylene 40 Stearate) | -- | -- |
| Sodium Phosphate Dibasic Heptahydrate | 0.268 | 0.268 |
| Citric Acid Monohydrate | 0.014 | 0.014 |
| Glycerin | 1.2 | 1.2 |
| Mannitol | 2.0 | 2.0 |
| IN NaOH/ IN HCl | 7.4 | 7.4 |
| Purified Water | q.s. to 100% | q.s. to 100% |

### Example 3 -- Improved BAK efficacy

Preservative titration studies were performed to compare the efficacy of benzalkonium chloride (BAK) in formulations using different solubilizers. Typically, it is seen that in the presence of surfactants, the preservative efficacy of BAK is significantly reduced. As a result, higher levels of BAK are required to meet the preservative criteria as defined in US and European Pharmacopeias. It was seen that when Solutol® HS 15 was used as a solubilizers, the preservative criteria were met at lower levels of BAK as compared to that using PS80, PS20, or POE40Sterate as summarized in Table 4.

**Table 4: Summary of Preservative Titration to Failure results for formulations containing different solubilizers. "Solutol" refers to Solutol® HS 15.**

| **BAK (ppm)** | **APET Criteria Met¹** | | | |
|---|---|---|---|---|
| | **1% PS80 F1 Solution Series*** | **1% Solutol F2 Solution Series** | **1% PS20 F3 Solution Series*** | **1% POE40 F4 Solution Series*** |
| 50 | USP | Ph Eur-B | USP | USP |
| 75 | Ph Eur-B | Ph Eur-B | Ph Eur-B | USP |
| 100 | Ph Eur-B | Ph Eur-A | Ph Eur-B | Ph Eur-B |
| 120 | Ph Eur-B | Ph Eur-A | Ph Eur-B | Ph Eur-B |
| 140 | Ph Eur-A | Ph Eur-A | Ph Eur-A | Ph Eur-B |
| 160 | Ph Eur-A | Ph Eur-A | Ph Eur-A | Ph Eur-B |
| Expt# | 22358 | 22839 | 22861 | 22861 |

| | | | | |
|---|---|---|---|---|
| ¹APET criteria as defined in USP and European Pharmacopeia (Ph Eur) * Comparative | | | | |

### Example 4 -- Tolerability for ophthalmic use

Formulations containing Solutol® HS 15 have been evaluated in rabbit ocular toxicology studies and were found to be well tolerated. Emulsions containing Solutol® HS 15 show improved tolerability vs those containing Polysorbate 80. Solution formulations containing Solutol® HS 15 at concentrations up to 2% show good tolerability in toxicology studies.

### Example 5 -- Formulations with Solutol® HS 15 for ophthalmic use

In view of the results herein, Solutol® HS 15 shows a variety of beneficial effects as a solubilizer for ophthalmic formulations. These include, but are not limited to, examples listed in Table 5. It may be used in formulations other than aqueous solutions as well.

Only the compositions containing 10-200 ppm of BAK are in accordance with the invention as claimed.

**Table 5: Prophetic Examples of Solution Formulations using Solutol® HS 15**

| **Ingredient type** | **Ingredient** | **Examples of typical conc. range % (w/w)** |
|---|---|---|
| **Active Ingredients** | | Any one of the below drug substances |
| Any one ofthe drug substances listed | Cyclosporine | 0.001-0.1% (e.g. about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.1%(w/w)) |
| | Phentolamine | 0.001-1% (e.g. about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1.0%(w/w)) |
| | Testosterone, and its derivatives | 0.001-5% (e.g. about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1,4.2, 4.3, 4.4, 4.5, 4.6, 4.7, 4.8, 4.9, or 5.0%(w/w)) |
| | Cmpd 1 | 0.0002-0.05%, 0.001-0.1%, 0.001-2.5% (e.g. about 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, or 2.5%(w/w)) |
| | Cmpd 2 | 0.0002-0.05%, 0.001-0.1%, 0.001-2.5% (e.g. about 0.0002, 0.0003, 0.0004, 0.0005, 0.0006, 0.0007, 0.0008, 0.0009, 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, or 2.5%(w/w)) |
| | Cmpd 3 | 0.0002-0.05%, 0.001-0.1%, 0.001-2.5% (e.g. about 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, or 2.5%(w/w)) |
| | Cmpd 4 | 0.0002-0.05%, 0.001-0.1%, 0.001-2.5% (e.g. about 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, or 2.5%(w/w)) |
| | Bimatoprost | 0.0002-0.05%, 0.001-0.1%, 0.001-2.5% (e.g. about 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, or 2.5%(w/w)) |
| **Solubilizer** | Solutol® HS 15 | 0.001-5% |
| **Secondary solubilizer/Co-solubilizer** | sorbitan stearate | 0-1% |
| | Pluronic® F68 | 0-5% |
| (may or may not be required) | POE40Stearate | 0-1% |
| | Cremophor® EL® | 0-1% |
| | Cyclodextrins | 0-10% |
| **Osmolality agents** | Propylene glycol | 0-2% |
| (any one or two or more in combinations) | Glycerin | 0-2.5% |
| | Mannitol | 0-5% |
| | Sodium chloride | 0-1% |
| **Buffers** | Phosphate buffer | 1-100 mM |
| (Any one ofthe buffers listed) | Phosphate citrate buffer | 1 -100 mM |
| | NaOH/Trolamine | 1-100 mM |
| | Lactate buffer | 1-100 mM |
| | Borate buffer | 1-100 mM |
| | Borate citrate | 1-100 Mm |
| | NaOH or HCl for pH adjustment | Q.S |
| **Preservatives** | None Non preserved | NA |
| (Any one or in combination) | BAK | 10-200 ppm |
| | Purite® | 10-300 ppm |
| | Water | QS |

### Example 6 -- Exemplary formulations

Exemplary formulations suitable for use in the compositions and methods described herein are set forth below.

**Table 7: Formulation composition of solutions containing the active Cmpd 2 and Polysorbate 80 and Solutol® HS 15, respectively as solubilizers.**

| Ingredients %w/w | | |
|---|---|---|
| Formula Name | PS-1 (comparative) | Sol-1 |
| Cmpd 2 | 0.0025 | 0.0025 |
| HPMC (F4M) | 1 | 1 |
| BAK | 0.012 | 0.012 |
| Polysorbate 80 | 0.05 | -- |
| Solutol HS 15 | -- | 0.05 |
| Citric Acid Monohydrate | 0.03 | 0.03 |
| Sodium Phosphate Dibasic Heptahydrate | 0.3 | 0.3 |
| Glycerin | 2.2 | 2.2 |
| Purified Water | q.s. to 100% | q.s. to 100% |

**Table 8: Formulation composition of solutions containing the active Cmpd 2 and Polysorbate 80 and Solutol® HS 15, respectively as solubilizers.**

| **Formulation Lot** # - | #**5 (comparative)** | **#2** |
|---|---|---|
| **Materials** | **%w/w** | **%w/w** |
| Cmpd 2 | 0.0015 | 0.0015 |
| Citric Acid Monohydrate | 0.03 | 0.03 |
| Sodium Phosphate Dibasic Heptahydrate | 0.3 | 0.3 |
| BAK | 0.01 | 0.01 |
| EDTA | 0.01 | 0.01 |
| Polysorbate 80 | 0.05 | 0 |
| Solutol HS 15 | 0 | 0.05 |
| Glycerin | 2.2 | 2.2 |
| Purified Water | q.s. to 100% | q.s. to 100% |

**Table 9: Cmpd 3 Formulation Stability Data**

| **Ingredient (% w/w)** | **Function** | **1% Solutol @pH 5.5** | **w/o Solutol @ pH 5.5*** | **1% PS80 @ pH 5.5*** | **1% Solutol @ pH 6.5** | **w/o Solutol @ pH 6.5*** |
|---|---|---|---|---|---|---|
| **Cmpd 3** | active | 0.0075 | 0.0075 | 0.0075 | 0.010 | 0.010 |
| Solutol HS 15 | solubilizer | 1.0 | -- | -- | 1.0 | -- |
| Polysorbate 80 | solubilizer | -- | -- | 1.0 | -- | -- |
| Glycerin | tonicity | 2.2 | 2.2 | 2.2 | 2.2 | 2.2 |
| BAK | preservatives | 0.01 | 0.01 | 0.01 | 0.01 | 0.01 |
| EDTA | chelating | 0.01 | 0.01 | 0.01 | 0.01 | 0.01 |
| Citric Acid Monohydrate | buffer | 0.1 | 0.1 | 0.1 | 0.052 | 0.052 |
| Sodium Phosphate Dibasic Heptahydrate | buffer | 0.32 | 0.32 | 0.32 | 0.32 | 0.32 |
| HCL/ NaOH, 1N | pH adjust | pH5.5 | pH5.5 | pH5.5 | pH 6.5 | pH 6.5 |

| Temperature | % Recovery at 1 month (n=1) | | | | | |
|---|---|---|---|---|---|---|
| **25°C** | 100.3 | | 96.9 | 96.2 | 96.6 | 96.0 |
| **40°C** | 95.3 | | 95.0 | 70.1 | 93.3 | 87.5 |
| **60°C** | 86.4 | | 86.3 | 16.3 | 81.6 | 58.4 |

| Temperature | % Recovery at 2 months (n=1) | | | | | |
|---|---|---|---|---|---|---|
| **25°C** | 101.6 | | 98.9 | 88.7 | 96.3 | 94.9 |
| **40°C** | 96.3 | | 97.2 | 22.5 | 90.3 | 80.1 |
| **60°C** | 70.1 | | 77.6 | 16.5 | 68.3 | 34.6 |

| Temperature | % Recovery at 3 months (n=1) | | | | | |
|---|---|---|---|---|---|---|
| **25°C** | 101.3 | | 97.1 | 78.6 | 97.4 | 95.1 |
| **40°C** | 94.0 | | 94.6 | 13.9 | 88.7 | 73.8 |
| **60°C** | 63.5 | | 66.0 | 9.9 | 61.4 | 19.1 |

| | | | | | | |
|---|---|---|---|---|---|---|
| * Comparative | | | | | | |

**Table 10: Cmpd 4 Solubility Data (reference example)**

| **Ingredient (% w/w)** | **Function** | | | | | | |
|---|---|---|---|---|---|---|---|
| **Cmpd 4** | active | saturate | saturate | saturate | saturate | saturate | saturate |
| Solutol® HS 15 | solubilizer | **0.1** | **0.5** | **1.0** | **0.1** | **0.5** | **1.0** |
| Glycerin | tonicity | 2.2 | 2.2 | 2.2 | 2.2 | 2.2 | 2.2 |
| Citric Acid Monohydrate | buffer | 0.108 | 0.108 | 0.108 | 0.052 | 0.052 | 0.052 |
| Sodium Phosphate Dibasic Heptahydrate | buffer | 0.32 | 0.32 | 0.32 | 0.32 | 0.32 | 0.32 |
| HCL/ NaOH, 1N | pH adjust | **pH5.5** | **pH5.5** | **pH5.5** | **pH 6.5** | **pH 6.5** | **pH 6.5** |
| | | | | | | | |
| **Cmpd 4 Solubility (% w/w)** <mixing overnights (>24 hr) at ambient room temperature> | | 0.07 | 0.11 | 0.16 | 0.02 | 0.06 | 0.10 |
| **Final pH** <post-filtration> | | 5.9 | 6.1 | 6.1 | 6.7 | 6.8 | 6.8 |

**Table 11: Bimatoprost Emulsion Formulation Compositions Stability Data**

| **Ingredient (% w/w)** | **Function** | **Emulsion with PS80 (comparative)** | **Emulsion with Solutol HS 15** |
|---|---|---|---|
| | | % w/w | % w/w |
| **Castor oil** | Oil phase | 0.1 | 0.1 |
| **Glycerin** | Tonicity agent | 2.2 | 2.2 |
| **EDTA** | Chelating agent | 0.01 | 0.01 |
| **BAK** | Preservative | 0.01 | 0.005 |
| **Bimatoprost** | API | 0.015 | 0.015 |
| **Polysorbate 80** | Emulsifier | 0.5 | -- |
| **Solutol 15 HS** | Emulsifier | -- | 0.125 |
| **POE-40-Sterate** | Secondary emulsifier | -- | 0.125 |
| **Sodium phosphate dibasic heptahydrate** | Buffer | 0.268 | 0.268 |
| **Citric acid monohydrate** | Buffer | 0.014 | 0.014 |
| **HPMC** | Viscosity agent | 0.25 | -- |
| **HEC** | Viscosity agent | -- | 0.25 |
| **Water** | Vehicle | qs 3L | qs 2L |
| **pH** | pH | 7.3 | 7.3 |

**Table 12: Bimatoprost Emulsion Formulation Stability Data at 40°C storage condition**

| **Formulation** | **Test** | **Results at timepoint** | | |
|---|---|---|---|---|
| | | **Initial** | **1 month** | **3 months** |
| **Emulsion with PS80 (comparative)** | **Bimatoprost assay (% of initial)** | 100.0 | 99.6 | 98.3 |
| | **Total impurities** | 0.55 | 0.71 | 1.59 |
| **Emulsion with Solutol HS 15** | **Bimatoprost assay (% of initial)** | 100.0 | 100.7 | 101.2 |
| | **Total impurities** | 0.41 | 0.23 | 0.18 |

### Example 7 -- Exemplary Active Pharmaceutical Ingredients

**Table 13: Active Pharmaceutical Ingredients**

| Compound, API | Structure | IUPAC name |
|---|---|---|
| Compound 1, Cmpd 1 | 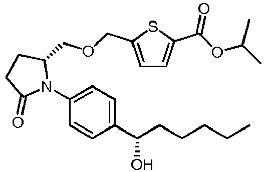 | isopropyl 5-((((R)-1-(4-((S)-1-hydroxyhexyl)phenyl)-5-oxopyrrolidin-2-yl)methoxy)methyl)thiophene-2-carboxylate |
| Compound 2, Cmpd2 | 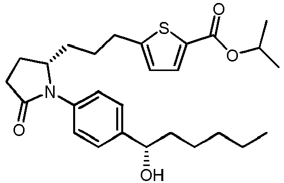 | isopropyl 5-(3-((S)-1-(4-((S)-1-hydroxyhexyl)phenyl)-5-oxopyrrolidin-2-yl)propyl)thiophene-2-carboxylate |
| Compound 3, Cmpd 3 | 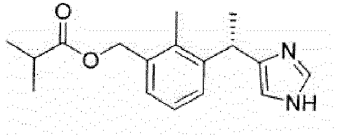 | 3-[(1S)-1-(1H-imidazol-4-yl)ethyl]-2-methylbenzyl 2-methylpropanoate |
| Compound 4, Cmpd 4 | 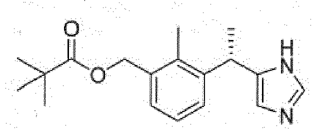 | 3-[(1S)-1-(1H-imidazol-4-yl)ethyl]-2-methylbenzyl pivalate |
| Bimatoprost | 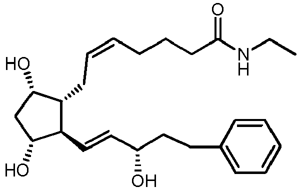 | (Z)-7-((1R,2R,3R,5S)-3,5-dihydroxy-2-((S,E)-3-hydroxy-5-phenylpent-1-enyl)cyclopentyl)-N-ethylhept-5-enamide |

### Example 8 -- Formulations with Solutol® HS 15 for treatment of corneal insensitivity due to corneal surgery

50 subjects age 18 to 40 who received corneal surgery (laser-assisted in situ deratomileusis/laser eye surgery) to correct myopia, hyperopia or astigmatism are randomly divided into 10 groups of 5 subjects each where each group is administered an ophthalmic composition from the list below (Example 15) on a schedule of one drop every 12 hours for two months. Corneal sensitivity is measured using the Cochet-Bonnet aesthesiometer (CBA) (Luneau, Paris, France) at the center of the cornea, where the cornea is most sensitive, at two, four, six, and eight weeks post-surgery to determine corneal sensitivity. Sensitivity measurements are conducted with the CBA filament first set to the longest length using a 0.12 mm diameter filament and reduced in length as required. Corneal nerve bundles are monitored by white light, tandem, slit scanning confocal microscopy prior to surgery and two, four, six, and eight weeks post-surgery. The cornea of each subject is scanned through its entire thickness and morphology and density of sub-basal nerve fiber bundles are being tabulated.

### Example 9 -- Formulations with Solutol® HS 15 for treatment of vernal keratoconjunctivitis

50 subjects age 18 to 30 suffering from vernal keratoconjunctivitis are randomly divided into 10 groups of 5 subjects each where each group is administered an ophthalmic composition from the list below (Example 15) on a schedule of one drop every 12 hours for one month. Efficacy of treatments include reduction in the presence of conjunctival papillae on the inside of eyelids, which are monitored by physical exam at beginning of treatment and at one, two, three, and four weeks. Reduction in damage to corneal surface is monitored by fluorescein eye stain at start of treatment and after one month. Additional measurements include hyperemia, chemosis, ocular mucous discharge, and ocular itching.

### Example 10 -- Formulations with Solutol® HS 15 for treatment of atopic keratoconjunctivitis

50 subjects age 18 to 30 suffering from atopic keratoconjunctivitis are randomly divided into 10 groups of 5 subjects each where each group is administered an ophthalmic composition from the list below (Example 15) on a schedule of one drop every 12 hours for one month. Efficacy of treatments include reduction in the presence of conjunctival papillae on the inside of eyelids, which are monitored by physical exam at beginning of treatment and at one, two, three, and four weeks. Reduction in damage to corneal surface is monitored by fluorescein eye stain at start of treatment and after one month. Additional measurements include hyperemia, chemosis, ocular mucous discharge, and ocular itching.

### Example 11 -- Formulations with Solutol® HS 15 for treatment of keratoconjunctivitis sicca or dry eye associated with keratoconjunctivitis sicca

50 subjects age 18 to 30 suffering from keratoconjunctivitis sicca or dry eye associated with keratoconjunctivitis sicca are randomly divided into 10 groups of 5 subjects each where each group is administered an ophthalmic composition from the list below (Example 15) on a schedule of one drop every 12 hours for one month. Efficacy of treatment is measured by monitoring the improvements in the physical state of the cornea (fluorescein eye stain with examination, slit lamp examination with and without flueorescein), volume of tear production and moisture on the surface of the eye (Schirmer's test with and without anesthesia using Whatman #41 filter paper), tear breakup time test using fluorescein, tear protein composition and analysis (lysozyme and Ap4A), and tear osmolarity test.

### Example 12 -- Formulations with Solutol® HS 15 for treatment of ocular inflammation

50 subjects age 18 to 65 suffering from ocular inflammation are randomly divided into 10 groups of 5 subjects each where each group is administered an ophthalmic composition from the list below (Example 15) on a schedule of one drop every 12 hours for 21 days. Improvements (reduction) in ocular inflammation are monitored by slit lamp examination to determine improvements in the cornea.

### Example 13 -- Formulations with Solutol® HS 15 for treatment of primary open angle glaucoma

50 subjects age 18 and older suffering from primary open angle glaucoma are randomly divided into 10 groups of 5 subjects each where each group is administered an ophthalmic composition from the list below (Example 15) on a schedule of one drop every 12 hours for 3 month. Exams are conducted every two weeks to determine efficacy of treatments. Efficacy of treatment is measured by monitoring intraocular pressure (tonometry), drainage angle of the eye (gonioscopy), peripheral vision (visual field test), visual acuity (visual test). The physical state of the optic nerve is monitored prior to treatment and post-treatment with fundus photographs being collected.

### Example 14 -- Formulations with Solutol® HS 15 for treatment of ocular hypertension

50 subjects age 18 and older suffering from ocular hypertension are randomly divided into 10 groups of 5 subjects each where each group is administered an ophthalmic composition from the list below (Example 15) on a schedule of one drop every 12 hours for 3 month. Intraocular pressure is monitored by tonometry every two weeks. Slit lamp examinations are conducted once a month to monitor the physical state of the cornea.

### Example 15 -- Formulations with macrogol 15 hydroxystearate for ophthalmic use in Examples 8 to 14 above.

| | |
|---|---|
| API (%(w/w) | Additional components (%(w/w)) |
| None | macrogol 15 hydroxystearate (1.0), glycerin (2.2), citric acid monohydrate (0.014), sodium phosphatedibasic heptahydrate (0.268), BAK (0.01), pH 7.4. |
| Cyclosporine (0.05) | macrogol 15 hydroxystearate (1.0), glycerin (2.2), citric acid monohydrate (0.014), sodium phosphatedibasic heptahydrate (0.268), BAK (0.01), pH 7.4. |
| Phentolamine (0.50) | macrogol 15 hydroxystearate (1.0), glycerin (2.2), citric acid monohydrate (0.014), sodium phosphatedibasic heptahydrate (0.268), BAK (0.01), pH 7.4. |
| Testosterone (0.03) | macrogol 15 hydroxystearate (1.0), glycerin (2.2), citric acid monohydrate (0.014), sodium phosphatedibasic heptahydrate (0.268), BAK (0.01), pH 7.4. |
| Testosterone derivative (0.03) | macrogol 15 hydroxystearate (1.0), glycerin (2.2), citric acid monohydrate (0.014), sodium phosphate dibasic heptahydrate (0.268), BAK (0.01), pH 7.4. |
| Cmpd 1 (0.075) | macrogol 15 hydroxystearate (1.0), glycerin (1.2), citric acid monohydrate (0.014), sodium phosphatedibasic heptahydrate (0.268), BAK (0.02), EDTA (0.01), mannitol (2.0), pH 7.4. |
| Cmpd 2(0.002) | macrogol 15 hydroxystearate (0.05), glycerin (2.2), citric acid monohydrate (0.03), sodium phosphatedibasic heptahydrate (0.3), BAK (0.012), HPMC (F4M) (1.0). |
| Cmpd 3 (0.01) | macrogol 15 hydroxystearate (1.0), glycerin (2.2), citric acid monohydrate (0.052), sodium phosphatedibasic heptahydrate (0.32), BAK (0.01), EDTA (0.01), pH 6.5. |
| Cmpd 4 (0.06) | macrogol 15 hydroxystearate (0.75), glycerin (2.2), citric acid monohydrate (0.052), sodium phosphatedibasic heptahydrate (0.32), BAK (0.012), pH 6.5. |
| Bimatoprost (0.03) | macrogol 15 hydroxystearate (0.125), castor oil (0.1), glycerin (2.2), EDTA (0.01), BAK (0.005), POE-40-sterate (0.125), sodium phosphate dibasic heptahydrate (0.268), citric acid monohydrate (0.014), HEC (0.25), pH 7.3. |

## Claims

1. An ophthalmic composition comprising an active pharmaceutical ingredient in an amount sufficient to contribute to the treatment, prevention or reduction of a symptom or symptoms of an ophthalmic disease or condition; macrogol 15 hydroxystearate; and benzalkonium chloride at a concentration of 10 to 200 ppm.

2. An ophthalmic composition according to Claim 1, wherein the macrogol 15 hydroxystearate is present at a concentration of 0.001 to 5% (w/w).

3. An ophthalmic composition according to Claim 1 or Claim 2, wherein the active pharmaceutical ingredient is selected from cyclosporine, phentolamine, testosterone, simenepag isopropyl, aganepag isopropyl, 3-[(1S)-1-(1H-imidazol-4-yl)ethyl]-2-methylbenzyl 2-methylpropanoate (Cmpd 3), 3-[(1S)-1-(1H-imidazol-4-yl)ethyl]-2-methylbenzyl pivalate (Cmpd 4), and bimatoprost.

4. An ophthalmic composition according to Claim 3, wherein the active pharmaceutical ingredient is selected from cyclosporine at a concentration of 0.001 to 0.1% (w/w), phentolamine at a concentration of 0.001 to 1.0% (w/w), testosterone at a concentration of 0.001 to 5.0% (w/w), simenepag isopropyl at a concentration of 0.001 to 2.5% (w/w), aganepag isopropyl at a concentration of 0.0002 to 2.5% (w/w), Cmpd 3 at a concentration of 0.001 to 2.5% (w/w), Cmpd 4 at a concentration of 0.001 to 2.5% (w/w), and bimatoprost at a concentration of 0.001 to 2.5% (w/w).

5. An ophthalmic composition according to any of Claims 1 to 4, which is an ointment, a cream, a microemulsion, an emulsion, or a composition comprising lipid nanoparticles.

6. An ophthalmic composition according to Claim 1, comprising
an active pharmaceutical ingredient selected from
cyclosporine at a concentration of 0.001-0.1% (w/w),
phentolamine at a concentration of 0.001-1.0% (w/w),
testosterone at a concentration of 0.001-5.0% (w/w),
simenepag isopropyl at a concentration of 0.001-0.1 % (w/w),
aganepag isopropyl at a concentration of 0.0002-0.05% (w/w),
Cmpd 3 at a concentration of 0.001-2.5% (w/w),
Cmpd 4 at a concentration of 0.001-2.5% (w/w), and
bimatoprost at a concentration of 0.001-2.5% (w/w);
macrogol 15 hydroxystearate at a concentration of 0.001-5% (w/w);
one or more osmolality agents selected from
propylene glycol at a concentration up to 2% (w/w),
glycerin at a concentration up to 2.5% (w/w),
mannitol at a concentration up to 5% (w/w), and
sodium chloride at a concentration up to 1% (w/w);
a buffer selected from
phosphate at a concentration of 1-100 mM,
phosphate citrate at a concentration of 1-100 mM,
sodium hydroxide/trolamine at a concentration of 1-100 mM,
lactate at a concentration of 1-100 mM,
borate at a concentration of 1-100 mM, and
borate citrate at a concentration of 1-100 mM; and
benzalkonium chloride at a concentration of 10-200 ppm

7. An ophthalmic composition according to Claim 1, consisting essentially of
an active pharmaceutical ingredient selected from
cyclosporine at a concentration of 0.001-0.1% (w/w),
phentolamine at a concentration of 0.001-1.0% (w/w),
testosterone at a concentration of 0.001-5.0% (w/w),
simenepag isopropyl at a concentration of 0.001-0.1 % (w/w),
aganepag isopropyl at a concentration of 0.0002-0.05% (w/w),
Cmpd 3 at a concentration of 0.001-2.5% (w/w),
Cmpd 4 at a concentration of 0.001-2.5% (w/w), and
bimatoprost at a concentration of 0.001-2.5% (w/w);
macrogol 15 hydroxystearate at a concentration of 0.001-5% (w/w);
one or more osmolality agents selected from
propylene glycol at a concentration up to 2% (w/w),
glycerin at a concentration up to 2.5% (w/w),
mannitol at a concentration up to 5% (w/w), and
sodium chloride at a concentration up to 1% (w/w);
a buffer selected from
phosphate at a concentration of 1-100 mM,
phosphate citrate at a concentration of 1-100 mM,
sodium hydroxide/trolamine at a concentration of 1-100 mM,
lactate at a concentration of 1-100 mM,
borate at a concentration of 1-100 mM, and
borate citrate at a concentration of 1-100 mM;
one or more secondary solubilizers selected from
sorbitan stearate at a concentration up to 1% (w/w),
polyoxyethylene-polyoxypropylene block copolymer at a concentration up to 5% (w/w),
polyoxyethylene 40 stearate at a concentration up to 1% (w/w),
polyethoxylated castor oil at a concentration up to 1% (w/w), and
one or more cyclodextrins at a concentration up to 10% (w/w); and
benzalkonium chloride at a concentration of 10-200 ppm.

8. An ophthalmic composition according to Claim 1, consisting of
an active pharmaceutical ingredient selected from
cyclosporine at a concentration of 0.001-0.1% (w/w),
phentolamine at a concentration of 0.001-1.0% (w/w),
testosterone at a concentration of 0.001-5.0% (w/w),
simenepag isopropyl at a concentration of 0.001-0.1 % (w/w),
aganepag isopropyl at a concentration of 0.0002-0.05% (w/w),
Cmpd 3 at a concentration of 0.001-2.5% (w/w),
Cmpd 4 at a concentration of 0.001-2.5% (w/w), and
bimatoprost at a concentration of 0.001-2.5% (w/w);
macrogol 15 hydroxystearate at a concentration of 0.001-5% (w/w);
one or more osmolality agents selected from
propylene glycol at a concentration up to 2% (w/w),
glycerin at a concentration up to 2.5% (w/w),
mannitol at a concentration up to 5% (w/w), and
sodium chloride at a concentration up to 1% (w/w);
a buffer selected from
phosphate at a concentration of 1-100 mM,
phosphate citrate at a concentration of 1-100 mM,
sodium hydroxide/trolamine at a concentration of 1-100 mM,
lactate at a concentration of 1-100 mM,
borate at a concentration of 1-100 mM, and
borate citrate at a concentration of 1-100 mM;
one or more secondary solubilizers selected from
sorbitan stearate at a concentration up to 1% (w/w),
polyoxyethylene-polyoxypropylene block copolymer at a concentration up to 5% (w/w),
polyoxyethylene 40 stearate at a concentration up to 1% (w/w), polyethoxylated castor oil at a concentration up to 1% (w/w), and
one or more cyclodextrins at a concentration up to 10% (w/w); and
benzalkonium chloride at a concentration of 10-200 ppm.

9. An ophthalmic composition according to any of Claims 1 to 8 for use in a method of treating a disease or disorder selected from ocular hypertension, primary open angle glaucoma, ocular inflammation, keratoconjunctivitis sicca, dry eye associated with keratoconjunctivitis sicca, vernel keratoconjunctivitis, atopic keratoconjunctivitis, and corneal insensitivity due to corneal surgery.

## Patentansprüche

1. Ophthalmische Zusammensetzung, die einen Pharmawirkstoff in einer Menge, die ausreichend ist für die Unterstützung der Behandlung, Prävention oder Verringerung von einem Symptom oder von Symptomen einer ophthalmischen Krankheit oder eines ophthalmischen Zustands; Macrogol-15 Hydroxystearat; und Benzalkoniumchlorid in einer Konzentration von 10 bis 200 ppm umfasst.

2. Ophthalmische Zusammensetzung gemäß Anspruch 1, wobei das Macrogol-15 Hydroxystearat in einer Konzentration von 0,001 bis 5 % (Gew./Gew.) vorhanden ist.

3. Ophthalmische Zusammensetzung gemäß Anspruch 1 oder Anspruch 2, wobei der Pharmawirkstoff ausgewählt ist aus Cyclosporin, Phentolamin, Testosteron, Simenepag Isopropyl, Aganepag Isopropyl, 3-[(1S)-1-(1H-Imidazol-4-yl)ethyl]-2-methylbenzyl-2-methylpropanoat (Cmpd 3), 3-[(1S)-1-(1H-Imidazol-4-yl)ethyl]-2-methylbenzylpivalat (Cmpd 4) und Bimatoprost.

4. Ophthalmische Zusammensetzung gemäß Anspruch 3, wobei der Pharmawirkstoff ausgewählt ist aus Cyclosporin in einer Konzentration von 0,001 bis 0,1 % (Gew./Gew.), Phentolamin in einer Konzentration von 0,001 bis 1,0 % (Gew./Gew.), Testosteron in einer Konzentration von 0,001 bis 5,0 % (Gew./Gew.), Simenepag Isopropyl in einer Konzentration von 0,001 bis 2,5 % (Gew./Gew.), Aganepag Isopropyl in einer Konzentration von 0,0002 bis 2,5 % (Gew./Gew.), Cmpd 3 in einer Konzentration von 0,001 bis 2,5 % (Gew./Gew.), Cmpd 4 in einer Konzentration von 0,001 bis 2,5 % (Gew./Gew.) und Bimatoprost in einer Konzentration von 0,001 bis 2,5 % (Gew./Gew.).

5. Ophthalmische Zusammensetzung gemäß mindestens einem der Ansprüche 1 bis 4, die eine Salbe, eine Creme, eine Mikroemulsion, eine Emulsion oder eine Zusammensetzung, die Nanolipidteilchen umfasst, ist.

6. Ophthalmische Zusammensetzung gemäß Anspruch 1, umfassend
einen Pharmawirkstoff ausgewählt aus
Cyclosporin in einer Konzentration von 0,001-0,1 % (Gew./Gew.),
Phentolamin in einer Konzentration von 0,001-1,0 % (Gew./Gew.),
Testosteron in einer Konzentration von 0,001-5,0 % (Gew./Gew.),
Simenepag Isopropyl in einer Konzentration von 0,001-0,1 % (Gew./Gew.),
Aganepag Isopropyl in einer Konzentration von 0,0002-0,05 % (Gew./Gew.),
Cmpd 3 in einer Konzentration von 0,001-2,5 % (Gew./Gew.),
Cmpd 4 in einer Konzentration von 0,001-2,5 % (Gew./Gew.), und
Bimatoprost in einer Konzentration von 0,001-2,5 % (Gew./Gew.);
Macrogol-15 Hydroxystearat in einer Konzentration von 0,001-5 % (Gew./Gew.);
ein oder mehr Osmolalitätsmittel ausgewählt aus
Propylenglykol in einer Konzentration von bis zu 2 % (Gew./Gew.),
Glycerin in einer Konzentration von bis zu 2,5 % (Gew./Gew.),
Mannitol in einer Konzentration von bis zu 5 % (Gew./Gew.), und
Natriumchlorid in einer Konzentration von bis zu 1 % (Gew./Gew.) ;
einen Puffer ausgewählt aus
Phosphat in einer Konzentration von 1-100 mM,
Phosphatcitrat in einer Konzentration von 1-100 mM,
Natriumhydroxid/Trolamin in einer Konzentration von 1-100 mM,
Lactat in einer Konzentration von 1-100 mM,
Borat in einer Konzentration von 1-100 mM, und
Boratcitrat in einer Konzentration von 1-100 mM;
und
Benzalkoniumchlorid in einer Konzentration von 10-200 ppm.

7. Ophthalmische Zusammensetzung gemäß Anspruch 1, die im Wesentlichen aus folgendem besteht:
einem Pharmawirkstoff ausgewählt aus
Cyclosporin in einer Konzentration von 0,001-0,1 % (Gew./Gew.),
Phentolamin in einer Konzentration von 0,001-1,0 % (Gew./Gew.),
Testosteron in einer Konzentration von 0,001-5,0 % (Gew./Gew.),
Simenepag Isopropyl in einer Konzentration von 0,001-0,1 % (Gew./Gew.),
Aganepag Isopropyl in einer Konzentration von 0,0002-0,05 % (Gew./Gew.),
Cmpd 3 in einer Konzentration von 0,001-2,5 % (Gew./Gew.),
Cmpd 4 in einer Konzentration von 0,001-2,5 % (Gew./Gew.), und
Bimatoprost in einer Konzentration von 0,001-2,5 % (Gew./Gew.);
Macrogol-15 Hydroxystearat in einer Konzentration von 0,001-5 % (Gew./Gew.);
einem oder mehreren Osmolalitätsmitteln ausgewählt aus
Propylenglykol in einer Konzentration von bis zu 2 % (Gew./Gew.),
Glycerin in einer Konzentration von bis zu 2,5 % (Gew./Gew.),
Mannitol in einer Konzentration von bis zu 5 % (Gew./Gew.), und
Natriumchlorid in einer Konzentration von bis zu 1 % (Gew./Gew.) ;
einem Puffer ausgewählt aus
Phosphat in einer Konzentration von 1-100 mM,
Phosphatcitrat in einer Konzentration von 1-100 mM,
Natriumhydroxid/Trolamin in einer Konzentration von 1-100 mM,
Lactat in einer Konzentration von 1-100 mM,
Borat in einer Konzentration von 1-100 mM, und
Boratcitrat in einer Konzentration von 1-100 mM;
einem oder mehreren sekundären Lösungsvermittlern ausgewählt aus
Sorbitanstearat in einer Konzentration von bis zu 1 % (Gew./Gew.),
Polyoxyethylen-Polyoxypropylen-Blockcopolymer in einer Konzentration von bis zu 5 % (Gew./Gew.),
Polyoxyethylen-40 Stearat in einer Konzentration von bis zu 1 % (Gew./Gew.),
polyethoxyliertem Kastoröl in einer Konzentration von bis zu 1 % (Gew./Gew.), und
einem oder mehreren Cyclodextrinen in einer Konzentration von bis zu 10 % (Gew./Gew.); und
Benzalkoniumchlorid in einer Konzentration von 10-200 ppm.

8. Ophthalmische Zusammensetzung gemäß Anspruch 1, bestehend aus:
einem Pharmawirkstoff ausgewählt aus
Cyclosporin in einer Konzentration von 0,001-0,1 % (Gew./Gew.),
Phentolamin in einer Konzentration von 0,001-1,0 % (Gew./Gew.),
Testosteron in einer Konzentration von 0,001-5,0 % (Gew./Gew.),
Simenepag Isopropyl in einer Konzentration von 0,001-0,1 % (Gew./Gew.),
Aganepag Isopropyl in einer Konzentration von 0,0002-0,05 % (Gew./Gew.),
Cmpd 3 in einer Konzentration von 0,001-2,5 % (Gew./Gew.),
Cmpd 4 in einer Konzentration von 0,001-2,5 % (Gew./Gew.), und
Bimatoprost in einer Konzentration von 0,001-2,5 % (Gew./Gew.);
Macrogol-15 Hydroxystearat in einer Konzentration von 0,001-5 % (Gew./Gew.);
einem oder mehreren Osmolalitätsmitteln ausgewählt aus
Propylenglykol in einer Konzentration von bis zu 2 % (Gew./Gew.),
Glycerin in einer Konzentration von bis zu 2,5 % (Gew./Gew.),
Mannitol in einer Konzentration von bis zu 5 % (Gew./Gew.), und
Natriumchlorid in einer Konzentration von bis zu 1 % (Gew./Gew.) ;
einem Puffer ausgewählt aus
Phosphat in einer Konzentration von 1-100 mM,
Phosphatcitrat in einer Konzentration von 1-100 mM,
Natriumhydroxid/Trolamin in einer Konzentration von 1-100 mM,
Lactat in einer Konzentration von 1-100 mM,
Borat in einer Konzentration von 1-100 mM, und
Boratcitrat in einer Konzentration von 1-100 mM;
einem oder mehreren sekundären Lösungsvermittlern ausgewählt aus
Sorbitanstearat in einer Konzentration von bis zu 1 % (Gew./Gew.),
Polyoxyethylen-Polyoxypropylen-Blockcopolymer in einer Konzentration von bis zu 5 % (Gew./Gew.),
Polyoxyethylen-40 Stearat in einer Konzentration von bis zu 1 % (Gew./Gew.),
polyethoxyliertem Kastoröl in einer Konzentration von bis zu 1 % (Gew./Gew.), und
einem oder mehreren Cyclodextrinen in einer Konzentration von bis zu 10 % (Gew./Gew.); und
Benzalkoniumchlorid in einer Konzentration von 10-200 ppm.

9. Ophthalmische Zusammensetzung gemäß mindestens einem der Ansprüche 1 bis 8 zur Verwendung in einem Verfahren zur Behandlung einer Krankheit oder Erkrankung ausgewählt aus okulärer Hypertension, primärem Offenwinkelglaukom, Augenentzündung, Keratokonjunktivitis sicca, trockenem Auge in Verbindung mit Keratokonjunktivitis sicca, Vernel-Keratokonjunktivitis, atopischer Keratokonjunktivitis und Hornhautunempfindlichkeit aufgrund einer Hornhautoperation.

## Revendications

1. Composition ophtalmique comprenant un principe actif pharmaceutique dans une quantité suffisante pour contribuer au traitement, à la prévention ou à la réduction d'un symptôme ou de symptômes d'une maladie ou pathologie ophtalmique; de l'hydroxystéarate de macrogol 15 ; et du chlorure de benzalkonium à une concentration de 10 à 200 ppm.

2. Composition ophtalmique selon la revendication 1, dans laquelle l'hydroxystéarate de macrogol 15 est présent à une concentration de 0,001 à 5 % (en poids).

3. Composition ophtalmique selon la revendication 1 ou la revendication 2, dans laquelle le principe actif pharmaceutique est sélectionné parmi la cyclosporine, la phentolamine, la testostérone, le simenepag isopropyl, l'aganepag isopropyl, le 3-[(1S)-1-(1H-imidazol-4-yl)ethyl]-2-methylbenzyl 2-methylpropanoate (Composé 3), le 3-[(1S)-1-(1H-imidazol-4-yl)ethyl]-2-methylbenzyl pivalate (Composé 4), et le bimatoprost.

4. Composition ophtalmique selon la revendication 3, dans laquelle le principe actif pharmaceutique est sélectionné parmi la cyclosporine à une concentration de 0,001 à 0,1 % (en poids), la phentolamine à une concentration de 0,001 à 1,0 % (en poids), la testostérone à une concentration de 0,001 à 5,0 % (en poids), le simenepag isopropyl à une concentration de 0,001 à 2,5 % (en poids), l'aganepag isopropyl à une concentration de 0,0002 à 2,5 % (en poids), le Composé 3 à une concentration de 0,001 à 2,5 % (en poids), le Composé 4 à une concentration de 0,001 à 2,5 % (en poids), et le bimatoprost à une concentration de 0,001 à 2,5 % (en poids).

5. Composition ophtalmique selon l'une quelconque des revendications 1 à 4, qui est une pommade, une crème, une microémulsion, une émulsion, ou une composition comprenant des nanoparticules lipidiques.

6. Composition ophtalmique selon la revendication 1, comprenant
un principe actif pharmaceutique sélectionné parmi
la cyclosporine à une concentration de 0,001 à 0,1 % (en poids),
la phentolamine à une concentration de 0,001 à 1,0 % (en poids),
la testostérone à une concentration de 0,001 à 5,0 % (en poids),
le simenepag isopropyl à une concentration de 0,001 à 0,1 % (en poids),
l'aganepag isopropyl à une concentration de 0,0002 à 0,05 % (en poids),
le Composé 3 à une concentration de 0,001 à 2,5 % (en poids),
le Composé 4 à une concentration de 0,001 à 2,5 % (en poids), et
le bimatoprost à une concentration de 0,001 à 2,5 % (en poids) ;
de l'hydroxystéarate de macrogol 15 à une concentration de 0,001 à 5 % (en poids) ;
un ou plusieurs agents d'osmolalité sélectionnés parmi
le propylène glycol à une concentration allant jusqu'à 2 % (en poids),
la glycérine à une concentration allant jusqu'à 2,5 % (en poids),
le mannitol à une concentration allant jusqu'à 5 % (en poids), et
le chlorure de sodium à une concentration allant jusqu'à 1 % (en poids) ;
un tampon sélectionné parmi
le phosphate à une concentration de 1 à 100 mM,
le citrate de phosphate à une concentration de 1 à 100 mM,
l'hydroxyde de sodium/la trolamine à une concentration de 1 à 100 mM,
le lactate à une concentration de 1 à 100 mM,
le borate à une concentration de 1 à 100 mM, et
le citrate de borate à une concentration de 1 à 100 mM ; et
du chlorure de benzalkonium à une concentration de 10 à 200 ppm.

7. Composition ophtalmique selon la revendication 1, consistant essentiellement en un principe actif pharmaceutique sélectionné parmi
la cyclosporine à une concentration de 0,001 à 0,1 % (en poids),
la phentolamine à une concentration de 0,001 à 1,0 % (en poids),
la testostérone à une concentration de 0,001 à 5,0 % (en poids),
le simenepag isopropyl à une concentration de 0,001 à 0,1 % (en poids),
l'aganepag isopropyl à une concentration de 0,0002 à 0,05 % (en poids),
le Composé 3 à une concentration de 0,001 à 2,5 % (en poids),
le Composé 4 à une concentration de 0,001 à 2,5 % (en poids), et
le bimatoprost à une concentration de 0,001 à 2,5 % (en poids) ;
de l'hydroxystéarate de macrogol 15 à une concentration de 0,001 à 5 % (en poids) ;
un ou plusieurs agents d'osmolalité sélectionnés parmi
le propylène glycol à une concentration allant jusqu'à 2 % (en poids), la glycérine à une concentration allant jusqu'à 2,5 % (en poids),
le mannitol à une concentration allant jusqu'à 5 % (en poids), et
le chlorure de sodium à une concentration allant jusqu'à 1 % (en poids) ;
un tampon sélectionné parmi
le phosphate à une concentration de 1 à 100 mM,
le citrate de phosphate à une concentration de 1 à 100 mM,
l'hydroxyde de sodium/la trolamine à une concentration de 1 à 100 mM,
le lactate à une concentration de 1 à 100 mM,
le borate à une concentration de 1 à 100 mM, et
le citrate de borate à une concentration de 1 à 100 mM ;
un ou plusieurs solubilisants secondaires sélectionnés parmi
le stéarate de sorbitane à une concentration allant jusqu'à 1 % (en poids),
le copolymère en bloc de polyoxyéthylène-polyoxypropylène à une concentration allant jusqu'à 5 % (en poids),
le stéarate de polyoxyéthylène 40 à une concentration allant jusqu'à 1 % (en poids),
l'huile de ricin polyéthoxylée à une concentration allant jusqu'à 1 % (en poids), et
une ou plusieurs cyclodextrines à une concentration allant jusqu'à 10 % (en poids) ; et
du chlorure de benzalkonium à une concentration de 10 à 200 ppm.

8. Composition ophtalmique selon la revendication 1, consistant en
un principe actif pharmaceutique sélectionné parmi
la cyclosporine à une concentration de 0,001 à 0,1 % (en poids),
la phentolamine à une concentration de 0,001 à 1,0 % (en poids),
la testostérone à une concentration de 0,001 à 5,0 % (en poids),
le simenepag isopropyl à une concentration de 0,001 à 0,1 % (en poids),
l'aganepag isopropyl à une concentration de 0,0002 à 0,05 % (en poids),
le Composé 3 à une concentration de 0,001 à 2,5 % (en poids),
le Composé 4 à une concentration de 0,001 à 2,5 % (en poids), et
le bimatoprost à une concentration de 0,001 à 2,5 % (en poids) ;
de l'hydroxystéarate de macrogol 15 à une concentration de 0,001 à 5 % (en poids) ;
un ou plusieurs agents d'osmolalité sélectionnés parmi
le propylène glycol à une concentration allant jusqu'à 2 % (en poids),
la glycérine à une concentration allant jusqu'à 2,5 % (en poids),
le mannitol à une concentration allant jusqu'à 5 % (en poids), et
le chlorure de sodium à une concentration allant jusqu'à 1 % (en poids) ;
un tampon sélectionné parmi
le phosphate à une concentration de 1 à 100 mM,
le citrate de phosphate à une concentration de 1 à 100 mM,
l'hydroxyde de sodium/la trolamine à une concentration de 1 à 100 mM,
le lactate à une concentration de 1 à 100 mM,
le borate à une concentration de 1 à 100 mM, et
le citrate de borate à une concentration de 1 à 100 mM ;
un ou plusieurs solubilisants secondaires sélectionnés parmi
le stéarate de sorbitane à une concentration allant jusqu'à 1 % (en poids),
le copolymère en bloc de polyoxyéthylène-polyoxypropylène à une concentration allant jusqu'à 5 % (en poids),
le stéarate de polyoxyéthylène 40 à une concentration allant jusqu'à 1 % (en poids),
l'huile de ricin polyéthoxylée à une concentration allant jusqu'à 1 % (en poids), et
une ou plusieurs cyclodextrines à une concentration allant jusqu'à 10 % (en poids) ; et
du chlorure de benzalkonium à une concentration de 10 à 200 ppm.

9. Composition ophtalmique selon l'une quelconque des revendications 1 à 8, pour une utilisation dans un procédé de traitement d'une maladie ou d'un trouble sélectionné parmi l'hypertension oculaire, le glaucome à angle ouvert primaire, l'inflammation oculaire, la kératoconjonctivite sèche, l'œil sec associé à la kératoconjonctivite sèche, la kératoconjonctivite vernale, la kératoconjonctivite atopique, et l'insensibilité cornéenne en raison d'une chirurgie cornéenne.
